# EUROPEAN PATENT APPLICATION

(11) **EP 2 316 841 A1**
(43) Date of publication of application: **04.05.2011**
(21) Application number: 11152192.8
(22) Date of filing: 18.04.2008
(51) Int. Cl.: C07F 9/38

(54) **Ibandronate sodium polymorphs**

(30) Priority: 19.04.2007 IN CH08492007; 26.02.2008 US 31488 P; 15.02.2008 IN CH04002008
(62) Divisional of application: 08746196.8
(71) Applicant: Dr. Reddy's Laboratories Limited, Hyderabad 500 016, Andhra Pradesh (IN); Dr. Reddy's Laboratories Inc., Bridgewater, NJ 08807 (US)
(72) Inventor: Vasamsetti, Satish Kumar, 530 040, Andhra Pradesh (IN); Devarakonda, Surya Narayana, 500 047, Andhra Pradesh (IN); Thaimattam, Ram, 500 029, Andhra Pradesh (IN); Raghupati, Balaji, 530 016, Andhra Pradesh (IN); Asnani, Minakshi, 500 072, Andhra Pradesh (IN); Rangineni, Srinivasulu, 509 102, Andhra Pradesh (IN); Muppidi, Vamsi Krishna, 507 303, Andhra Pradesh (IN)
(74) Representative: Bates, Philip Ian

(57) **Abstract**

A crystalline form Alpha of ibandronate sodium and process for production thereof.

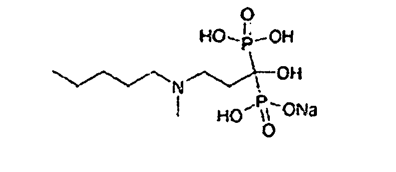

## Description

The present application relates to new crystalline forms of ibandronate sodium and processes for preparation thereof.

### BACKGROUND

Ibandronate sodium is a nitrogen-containing bisphosphonate that inhibits osteoclast-mediated bone resorption. The chemical name of ibandronate sodium is 3-(N-methyl-N-pentyl) amino-1-hydroxypropane-1,1-diphosphonic acid monosodium salt, represented by the chemical structure of Formula (I),

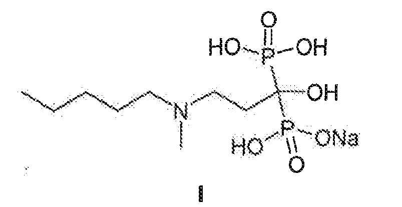

The monosodium salt of Ibandronic acid is useful in the treatment of bone disorders such as hypocalcaemia of malignance, osteolysis, Paget's disease, osteoporosis and metastatic bone diseases and is available in the market under the trade name Boniva™. It is indicated for the treatment and prevention of osteoporosis in postmenopausal women.

Gall et al in US 4,927,814 describes Ibandronic acid and its analogues generically and specifically, physiologically acceptable salts thereof. It also discloses a pharmaceutical composition and its use in the treatment of prophylaxis of calcium metabolism disturbance or disease.

Eiermann et al in WO 2006081963 describes a crystalline form of ibandronate sodium monohydrate, designated as crystalline Form A and process for its preparation.

Eiermann et al in another PCT application, WO 2006081962 describes another crystalline form of ibandronate sodium monohydrate, designated as crystalline Form B along with process for its preparation.

Lifshitz-Liron et al in WO 2006024024 describes several crystalline forms of ibandronate sodium designated as Forms C, D, E, F, G, H, J, K, K2, K3, Q, Q1, Q2, Q3, Q4, Q5, Q6, QQ, R, S, T, hemi-ethanolate and ethanolate, along with an amorphous form and processes for preparation thereof.

Pulla Reddy Muddasani et al in WO 2007074475 describes new crystalline forms of ibandronate sodium monohydrate designated as Forms I and II. The application also describes amorphous ibandronate sodium monohydrate.

Even though, all the above patents and patent applications discloses various polymorphic forms, however, there is still exist a need of novel crystalline forms, which may be used for the commercial manufacturing, and may yield both formulation and therapeutic benefits.

Different forms may provide different properties, such as solubility, which could be important in dosage form design and in terms of bioavailability and/or bioequivalence. Different forms may also provide stability advantages. Since polymorphic forms can vary in their physical properties, regulatory authorities require that efforts be made to identify all polymorphic forms, *e.g*., crystalline, amorphous, and pseudopolymorphic forms, *e.g.* solvates, etc., of new drug substances.

Some polymorphs of drug substances suffer from the drawbacks of conversion to other crystalline forms on storage resulting in concomitant change, not only in the physical form and shape of the drug crystals, but also the associated changes in distinct physical properties. Generally, the molecules will revert to a more thermodynamically stable form, often a form with lower solubility. Such a thermodynamically stable form may sometimes result in a reduced or suboptimal bioavailability, especially for oral administration.

Towards this end, it has been the endeavor of pharmaceutical scientists to provide new crystalline forms of the drug substances, more specifically, thermodynamically stable forms of drug substances, which would have the strengths of the crystalline forms, viz. thermodynamic stability, viz. enhanced solubility, rapid onset of action and an enhanced bioavailability.

### SUMMARY

The present application provides crystalline forms of ibandronate sodium and processes for preparing thereof.

In one aspect, there is provided a crystalline form of ibandronate sodium designated as Form I, and characterized by X-ray powder diffraction pattern with characteristic peaks at about 5.2, 17.4, 20.1, 25.2, and 31.3 ±0.2 degrees two theta.

In another aspect, there is provided another crystalline form of ibandronate sodium designated as Form II, characterized by X-ray powder diffraction pattern with characteristic peaks at about 5.2, 10.6, 17.2, 18.1, 21.6, 25.6, and 33.6±0.2 degrees two theta.

In another aspect, there is provided another crystalline form of ibandronate sodium designated as Form III, characterized by X-ray powder diffraction pattern with characteristic peaks approximately at: 5.4, 10.9, 14.4, 17.3, 18.2, 19.4, 20.3, 21.7, 24.7 and 25.7 ± 0.2 degrees two theta.

In another aspect, there is provided another crystalline form of ibandronate sodium designated as Form IV, characterized by X-ray powder diffraction pattern with characteristic peaks at about 4.9, 9.7, 19.7, 21.2, 21.9, 26.5, and 31.1 ± 0.2degrees two theta.

In another aspect, there is provided another crystalline form of ibandronate sodium designated as Form V, characterized by X-ray powder diffraction pattern with characteristic peaks at about 4.8, 10.8,19.7, 22.0, and 31.1 ± 0.2 degrees two theta.

In another aspect, there is provided another crystalline form of ibandronate sodium designated as Form VI, characterized by X-ray powder diffraction pattern with characteristic peaks at about 4.8, 9.6, 15.4, 19.4, 21.7, 24.2, 28.2 32.9, and 37.7 ± 0.2 degrees two theta.

In another aspect, there is provided another crystalline form of crystalline ibandronate sodium designated as Form VII, characterized by X-ray powder diffraction pattern with characteristic peaks at about 4.9, 9.8, 10.9, 14.1, 17.1, 18.6, 19.8, 23.8, 24.8, and 25.9 ± 0.2 degrees two theta.

In another aspect, there is provided another crystalline form of ibandronate sodium designated as Form VIII, characterized by X-ray powder diffraction pattern with characteristic peaks at about 6.1, 16.7, 18.1, 20.3, and 30.2 ± 0.2 degrees two theta.

In another aspect, there is provided another Crystalline form of ibandronate sodium designated as Form IX characterized by X-ray powder diffraction pattern with characteristic peaks at about 19.5, 20.8, 25.4, 26.3 and 34.9± 0.2degrees two theta.

In another aspect, there is provided another crystalline form of ibandronate sodium designated as Form X characterized by X-ray powder diffraction pattern with characteristic peaks at about 4.6, 5.8, 17.2, 19.4, 24.4 and 28.0± 0.2degrees two theta.

In another aspect, there is provided another crystalline form of ibandronate sodium designated as Form XI characterized by X-ray powder diffraction pattern with characteristic peaks at about 4.7, 5.9, 9.6, 17.1, and 19.4 ±0.2degrees two theta.

In another aspect, there is provided another crystalline form of ibandronate sodium designated as Form XII characterized by X-ray powder diffraction pattern with characteristic peaks at about 4.9, 6.0, 9.7, 12.4, 17.0, 19.3, 24.9, 29.3, 30.3 and 36.4 ± 0.2degrees two theta.

In another aspect, there is provided another crystalline form of ibandronate sodium designated as Form XIII characterized by X-ray powder diffraction pattern with characteristic peaks at about 4.8, 6.0, 9.7, 16.9, 19.7, 24.9 and 31.1 ± 0.2degrees two theta.

In another aspect, there is provided another crystalline form of ibandronate sodium designated as Form XIV characterized by X-ray powder diffraction pattern with characteristic peaks at about 4.8, 6.0, 9.6, 17.0, 18.2, 19.2, 20.1, and 24.8 ± 0.2degrees two theta.

In another aspect, there is provided another crystalline form of ibandronate sodium designated as Form XV characterized by X-ray powder diffraction pattern with characteristic peaks at about 4.7, 5.9, 9.6, 19.5, 21.4, 26.3, 30.8, and 35.7 ± 0.2degrees two theta.

In another aspect, there is provided another crystalline form of ibandronate sodium designated as Form XVI characterized by X-ray powder diffraction pattern with characteristic peaks at about 5.0, 5.9, 10.1, 15.2, 15.7, 16.1, 17.0, 20.0, 22.4, and 23.8 ± 0.2degrees two theta.

In another aspect, there is provided another crystalline form of ibandronate sodium designated as Form XVII characterized by X-ray powder diffraction pattern with characteristic peaks at about 4.5, 5.8, 8.9, 18.1, 19.8, 24.5, 25.9, 29.6 and 35.5 ± 0.2degrees two theta.

In another aspect, the present invention provides another crystalline form of ibandronate sodium designated as Form XVIII characterized by X-ray powder diffraction pattern with characteristic peaks at about 5.0, 5.9, 10.0, 17.0, 20.1, and 28.1 ± 0.2degrees two theta.

In another aspect, there is provided another crystalline form of ibandronate sodium designated as Form XIX characterized by X-ray powder diffraction pattern with characteristic peaks at 4.7, 5.7, 9.4, 17.0, 21.4, 24.3, 28.2, 29.5, and 35.4 ± 0.2 degrees two theta.

In another aspect, there is provided another crystalline form of ibandronate sodium designated as Form XX characterized by X-ray powder diffraction pattern with characteristic peaks at about 5.1, 5.8, 16.7, 20.8, 25.0, and 33.3 ± 0.2degrees two theta.

In another aspect, there is provided another crystalline form of ibandronate sodium designated as Form XXI characterized by X-ray powder diffraction pattern with characteristic peaks at about 4.6, 5.9, 16.4, 20.2, 24.7, 26.2, 28.3, 29.7 and 35.6 ± 0.2degrees two theta.

In another aspect, there is provided another crystalline form of ibandronate sodium designated as Form XXII characterized by X-ray powder diffraction pattern with characteristic peaks at about 5.2, 17.2, 19.4, 20.2, and 25.6 ± 0.2degrees two theta.

In another aspect, there is provided another crystalline form of ibandronate sodium designated as Form XXIII characterized by X-ray powder diffraction pattern with characteristic peaks at about 4.7, 12.7, 17.1, 17.5, 19.2, and 28.3 ± 0.2degrees two theta.

In another aspect, there is provided another crystalline form of ibandronate sodium designated as Form XXIV characterized by X-ray powder diffraction pattern with characteristic peaks at about 4.6, 9.1, 17.3, 18.5, and 19.7 ± 0.2 degrees two theta.

In another aspect, there is provided another Crystalline form of ibandronate sodium designated as Form XXV characterized by X-ray powder diffraction pattern with characteristic peaks at about 4.6, 5.2, 10.6, 13.0, 17.1, 19.4, 20.2 and 34.6 ± 0.2degrees two theta.

In another aspect, there is provided another crystalline form of ibandronate sodium designated as Form XXVI characterized by X-ray powder diffraction pattern with characteristic peaks at 3.8, 4.5, 9.0, 9.9, 17.3, 18,4 and 19.8 ± 0.2degrees two theta.

In another aspect, there is provided another crystalline form of ibandronate sodium designated as Form XXVII characterized by X-ray powder diffraction pattern with characteristic peaks approximately at: 4.5, 5.1, 10.3, 15.4,17.1, 19.5, 20.7, 25.3, 26.4 and 30.6 ± 0.2degrees two theta.

In another aspect, there is provided another crystalline form of ibandronate sodium designated as Form XXVIII characterized by X-ray powder diffraction pattern with characteristic peaks at 4.4, 5.9, 9.7, 12.1, 17.1, 19.4, 21.3, 24.9, 30.3 and 35.8 ± 0.2degrees two theta.

In another aspect, there is provided another crystalline form of ibandronate sodium designated as Form XXIX characterized by X-ray powder diffraction pattern with characteristic peaks at 6.0, 6.3, 12.4, 14.3, 16.5, 19.7, 20.2, 21.5, 24.7, and 26.2 ± 0.2degrees two theta.

In another aspect, there is provided another crystalline form of ibandronate sodium designated as Form XXX characterized by X-ray powder diffraction pattern with characteristic peaks at about 4.7, 5.1, 5.7, 14.0, 16.2, 20.0, 21.1, 24.5, 25.9, and 28.0 ± 0.2degrees two theta.

In another aspect, there is provided another crystalline form of ibandronate sodium designated as Form XXXI characterized by X-ray powder diffraction pattern with characteristic peaks at about 4.5, 4.7, 8.9, 13.4, and 26.6 ± 0.2degrees two theta.

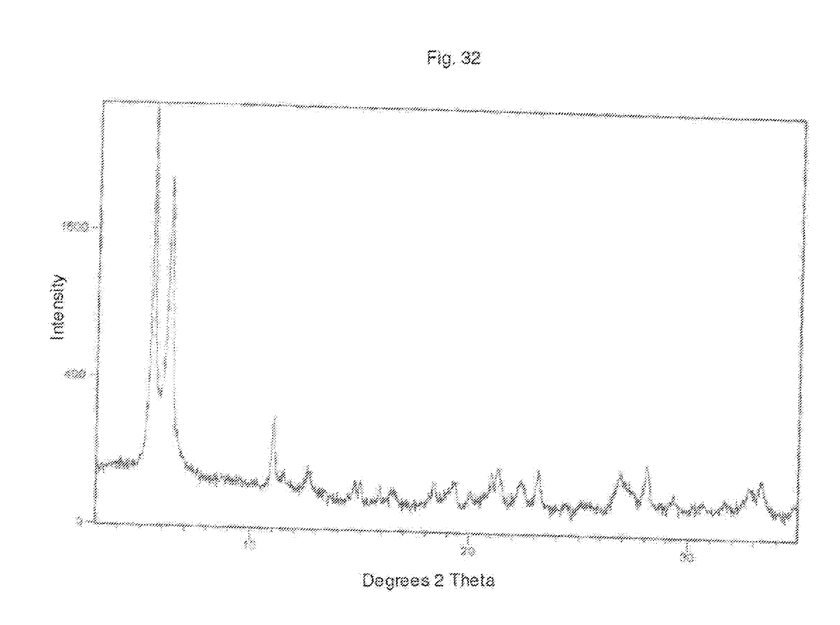

In yet another aspect, there is provided another crystalline form of ibandronate sodium designated as Form Alpha characterized by X-ray powder diffraction pattern with characteristic peaks at about 5.5, 6.3, 19.3, and 23.1 ±0.2 degrees two theta, which is substantially in accordance with the Figure 32.

In yet further another aspect there is provided another crystalline form of ibandronate sodium designated as Form Beta characterized by X-ray powder diffraction pattern with characteristic peaks at about 5.5, 10.9, 19.3, 23.1 and 33.2 ±0.2 degrees two theta.

In another aspect, there is provided a pharmaceutical composition comprising crystalline forms of ibandronate sodium of the present invention with pharmaceutically acceptable excipient.

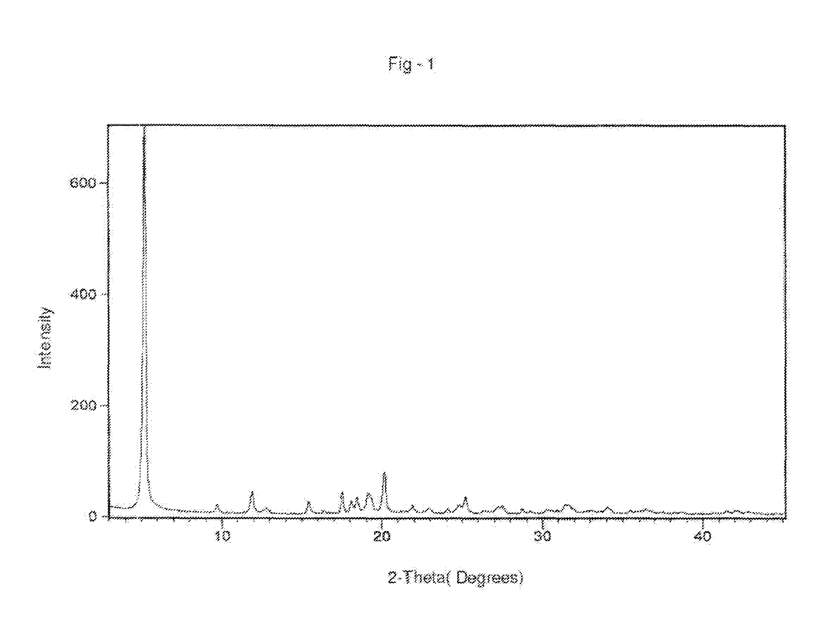

In another embodiment, the present invention includes the unique crystalline forms of ibandronate sodium identified herein as I-XXXI, alpha and beta having the patterns as substantially shown or depicted in Figures 1-33. These are also referred to herein as crystalline forms having an X-ray powder diffraction pattern substantially as depicted in a particular figure. By "substantially" it will be appreciated that patterns can be shifted in their peak positions and relative peak intensifies due to a number of factors known to those of skill in the crystallographic and powder X-ray diffraction arts including, without limitation: the equipment used, the sample preparation, preferred packing and orientations, the radiation source, and the like. However, those of ordinary skill in the art should be able to compare the figures herein with a pattern generated of an unknown form of ibandronate sodium and confirm its identity as one of the forms disclosed and claimed herein.

In still another embodiment, the present invention includes a number of crystalline forms of ibandronate sodium which can be designated as Forms I-XXXI and alpha and beta and which can be described individually by selecting any number of peaks, often between 4 and 10, which + 0.2 degrees two theta, uniquely identify that form.

In still another embodiment, the present invention includes a number of crystalline forms of ibandronate sodium which can be designated as Forms I, V, VIII, alpha and beta. In still another embodiment in accordance with the present invention, there is provided a crystalline ibandronate sodium solvate, also referred to herein as a "solvatomorph." Solvates of ibandronate sodium in accordance with the present invention include those produced using water, hydrocarbon solvents, ketones, alcohols, nitriles, ethers, amines, esters and acids. In particular, crystalline ibandronate solvates are those produced using n-hexane, n-heptane, cyclohexane, toluene, xylene, acetone, n-butanone, methyl isobutyl ketone, ethyl methyl ketone, acetone, acetonitrile, propionitrile, methanol, ethanol, isopropyl alcohol, n-propanol, t-butyl alcohol, n-butanol, sec- butanol, dimethyl sulfoxide, methyl tertiary butyl ether, dichloromethane, formamide, diethyl ether, diethyl ether, diisopropyl ether, tetrahydrofuran, acetic acid, formic acid, citric acid, succinic acid, ethyl acetate, dimethylformamide, or dimethylacetamide, Thus, for example, a solvate could be an ibandronate sodium formic acid solvate. Solvates include any proportion of solvent (not free solvent - bound solvent, also known as solvent of crystallization) to the underlying ibandronate sodium molecule such as hemisolvates, monosolvates, sesquasolvates, disolvates, trisolvates, tetrasolvates, pentasolvates, and the like. Form V, for example, is a formic acid solvate (1:1).

Another aspect of the present invention is a process for the preparation of crystalline Form Alpha. The process includes the desolvation of a solvate of ibandronate sodium until the residual solvent content is less than about 1% weight/weight. This can be accomplished by drying. In another embodiment, Form Alpha can be produced by desolvating a formic acid solvate, an acetic acid solvate, an ethanol glycol solvate, or a dimethyl sulfoxide ("DMSO") solvate by desolvating the solvate until the residual solvent content is less than about 1% weight/weight. Ibandronate sodium solvate can be desolvated by drying at an appropriate temperature for a time sufficient to obtain the desired reduction in solvent content.

Drying can be accomplished at a temperature of between about 50 and about 150 degrees C.

In another aspect of the present invention, a process is provided for converting crystalline Form Alpha to crystalline Form Beta ibandronate sodium comprising exposing Form Alpha to a humid environment for a time sufficient to accomplish conversion. Preferably, the moisture content of the atmosphere used is more than 30%. More preferably, the humid environment contains a moisture content in the range of 40 to 80%.

### BRIEF DESCRIPTION OF THE DRAWINGS

Figure 1: Illustrative example of X-ray powder diffraction (XRPD) pattern of ibandronate sodium crystalline Form I of Example 2.
Figure 2: Illustrative example of X-ray powder diffraction (XRPD) pattern of ibandronate sodium crystalline Form II of Example 8.
Figure 3: Illustrative example of X-ray powder diffraction (XRPD) pattern of ibandronate sodium crystalline Form III of Example 9.
Figure 4: Illustrative example of X-ray powder diffraction (XRPD) pattern of ibandronate sodium crystalline Form IV of Example 12.
Figure 5: Illustrative example of X-ray powder diffraction (XRPD) pattern of ibandronate sodium crystalline Form V of Example 14.
Figure 6: Illustrative example of X-ray powder diffraction (XRPD) pattern of ibandronate sodium crystalline Form VI of Example 16.
Figure 7: Illustrative example of X-ray powder diffraction (XRPD) pattern of ibandronate sodium crystalline Form VII of Example 17.
Figure 8: Illustrative example of X-ray powder diffraction (XRPD) pattern of ibandronate sodium crystalline Form VIII of Example 19.
Figure 9: Illustrative example of X-ray powder diffraction (XRPD) pattern of ibandronate sodium crystalline Form IX of Example 20.
Figure 10: Illustrative example of X-ray powder diffraction (XRPD) pattern of ibandronate sodium crystalline Form X of Example 21.
Figure 11: Illustrative example of X-ray powder diffraction (XRPD) pattern of ibandronate sodium crystalline Form XI of Example 22.
Figure 12: Illustrative example of X-ray powder diffraction (XRPD) pattern of ibandronate sodium crystalline Form XII of Example 23.
Figure 13: Illustrative example of X-ray powder diffraction (XRPD) pattern of ibandronate sodium crystalline Form XIII of Example 24.
Figure 14: Illustrative example of X-ray powder diffraction (XRPD) pattern of ibandronate sodium crystalline Form XIV of Example 25.
Figure 15: Illustrative example of X-ray powder diffraction (XRPD) pattern of ibandronate sodium crystalline Form XV of Example 26.
Figure 16: Illustrative example of X-ray powder diffraction (XRPD) pattern of ibandronate sodium crystalline Form XVI of Example 27.
Figure 17: Illustrative example of X-ray powder diffraction (XRPD) pattern of ibandronate sodium crystalline Form XVII of Example 28.
Figure 18: is an X-ray powder diffraction (XRPD) pattern of ibandronate sodium crystalline Form XVIII of Example 29.
Figure 19: Illustrative example of X-ray powder diffraction (XRPD) pattern of ibandronate sodium crystalline Form XIX of Example 30.
Figure 20: Illustrative example of X-ray powder diffraction (XRPD) pattern of ibandronate sodium crystalline Form XX of Example 31.
Figure 21: Illustrative example of X-ray powder diffraction (XRPD) pattern of ibandronate sodium crystalline Form XXI of Example 32.
Figure 22: Illustrative example of X-ray powder diffraction (XRPD) pattern of ibandronate sodium crystalline Form XXII of Example 33.
Figure. 23: Illustrative example of X-ray powder diffraction (XRPD) pattern of ibandronate sodium crystalline Form XXIII of Example 34.
Figure 24: Illustrative example of X-ray powder diffraction (XRPD) pattern of ibandronate sodium crystalline Form XXIV of Example 35.
Figure 25: Illustrative example of X-ray powder diffraction (XRPD) pattern of ibandronate sodium crystalline Form XXV of Example 36.
Figure 26: Illustrative example of X-ray powder diffraction (XRPD) pattern of ibandronate sodium crystalline Form XXVI of Example 37.
Figure 27: Illustrative example of X-ray powder diffraction (XRPD) pattern of ibandronate sodium crystalline Form XXVII of Example 38.
Figure 28: Illustrative example of X-ray powder diffraction (XRPD) pattern of ibandronate sodium crystalline Form XXVIII of Example 39.
Figure 29: Illustrative example of X-ray powder diffraction (XRPD) pattern of ibandronate sodium crystalline Form XXIX of Example 40.
Figure 30: Illustrative example of X-ray powder diffraction (XRPD) pattern of ibandronate sodium crystalline Form XXX of Example 41,
Figure 31: Illustrative example of X-ray powder diffraction (XRPD) pattern of ibandronate sodium crystalline Form XXXI of Example 42.
Figure 32: Illustrative example of X-Ray Powder Diffraction pattern of an illustrative sample of ibandronate sodium crystalline Form Alpha of Example 43,
Figure 33: Illustrative example of X-Ray Powder Diffraction pattern of an illustrative sample of ibandronate sodium crystalline Form Beta of Example 44.

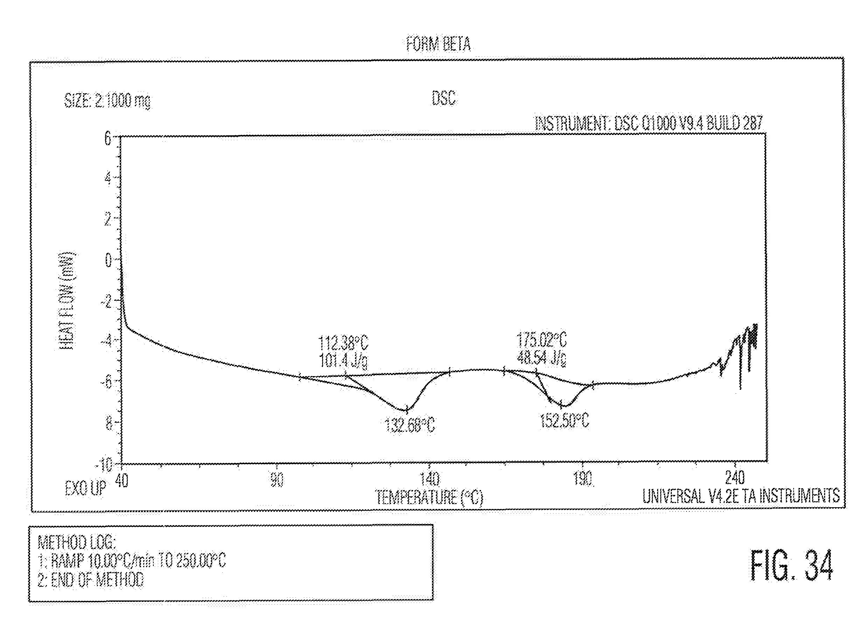

Figure 34: Illustrates the DSC for Form Beta run at 10°C/min to 250°C.

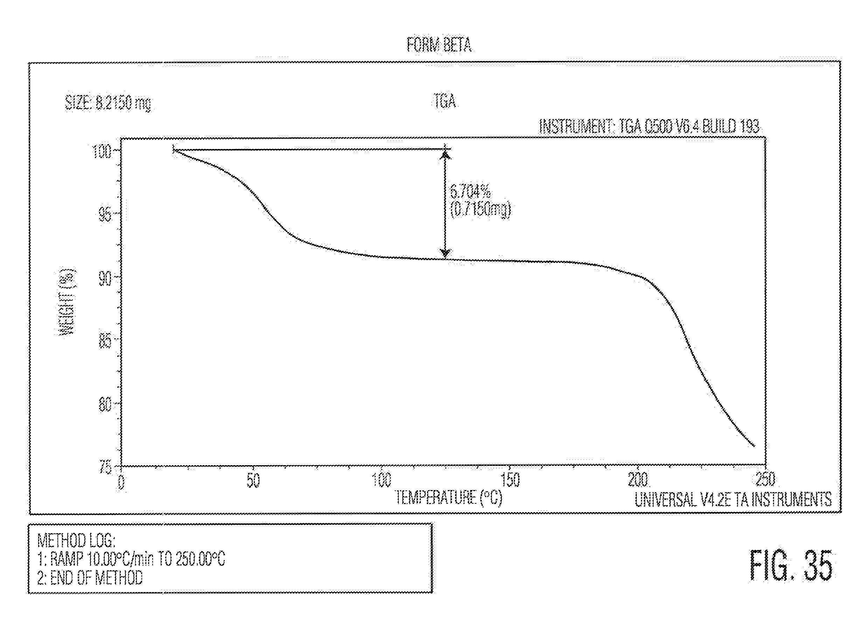

Figure 35: Illustrates the TGA for Form Beta run at 10°C/min to 250°C.

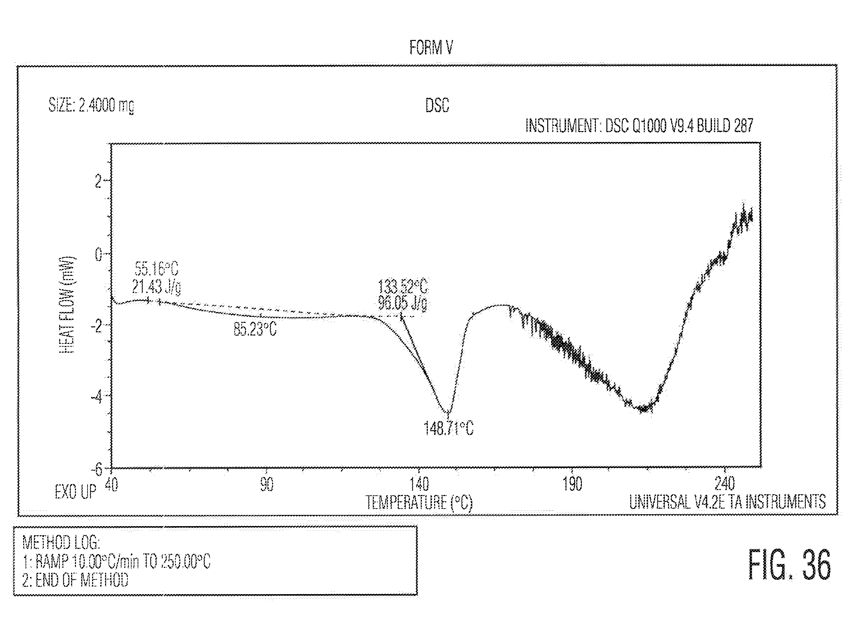

Figure 36: Illustrates the DSC for Form V run at 10°C/min to 250°C.

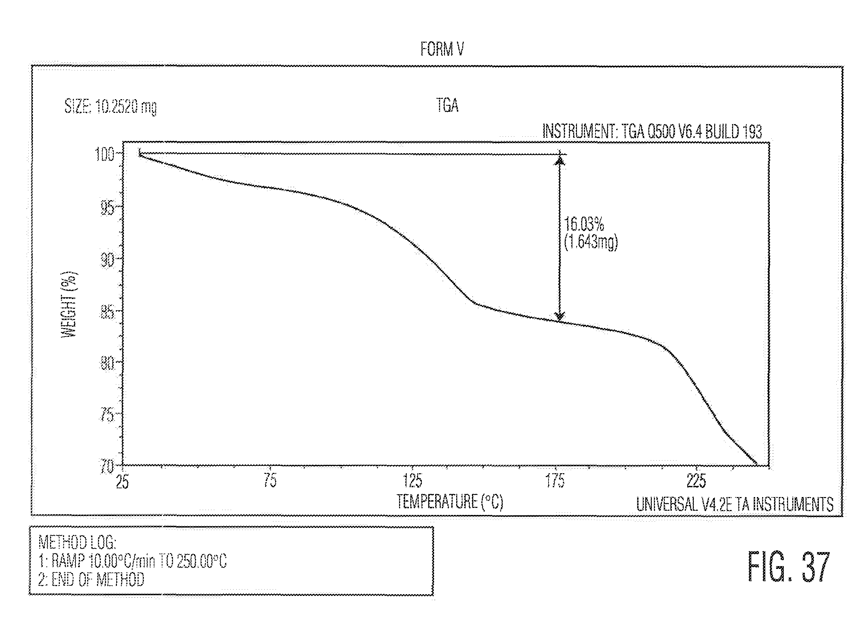

Figure 37: Illustrates the TGA for Form V run at 10°C/min to 250°C.

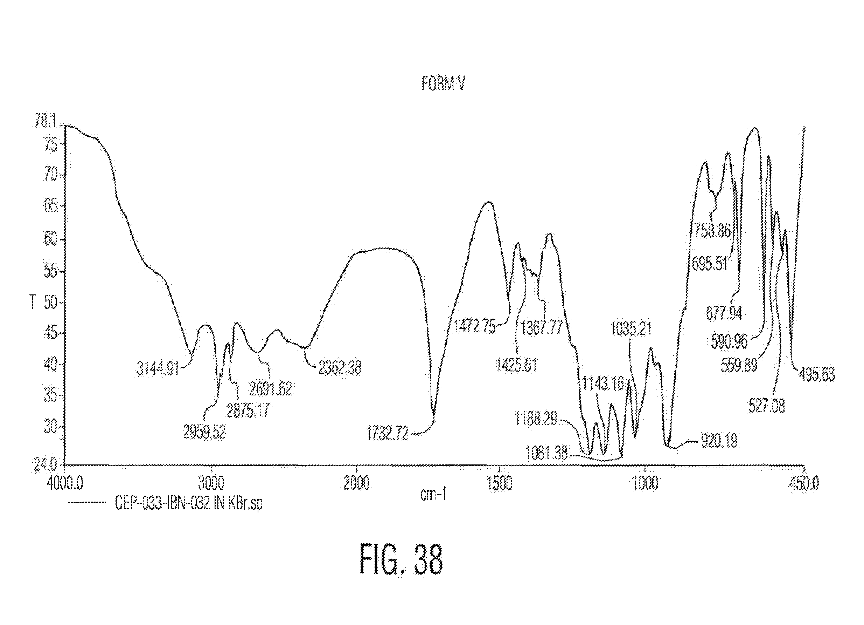

Figure 38: Illustrates the IR spectra for Form V.

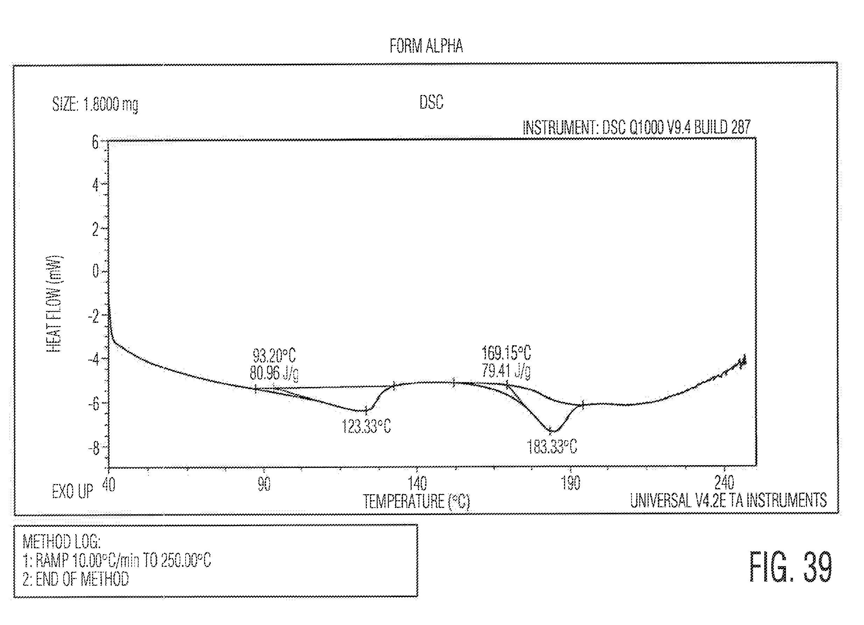

Figure 39: Illustrates the DSC for Form Alpha run at 10°C/min to 250°C.

### DETAILED DESCRIPTION

The present application provides crystalline forms of ibandronate sodium and process for preparing thereof.

In one aspect, there is provided a novel crystalline form of ibandronate sodium designated as Form 1, characterized by X-ray powder diffraction pattern with characteristic peaks at about 5.2, 17.4, 20.1, 25.2, and 31.3 ± 0.2 degrees two theta. In addition to or instead of any of the characteristic peaks described herein above, the X-ray powder diffraction pattern may also include peaks at 11.9, 15.4, 18.0, 18.4, 19.1, 19.3, 20.1, 21.9, 24.7, and 31.3 ±0.2 degrees. It should be kept in mind that XRPD patterns for the same solid form typically vary as a function of a number of relevant factors, some of which include X-ray diffraction equipment and operator-to-operator variability. Figure 1 shows a representative X-ray powder diffraction pattern for Form I.

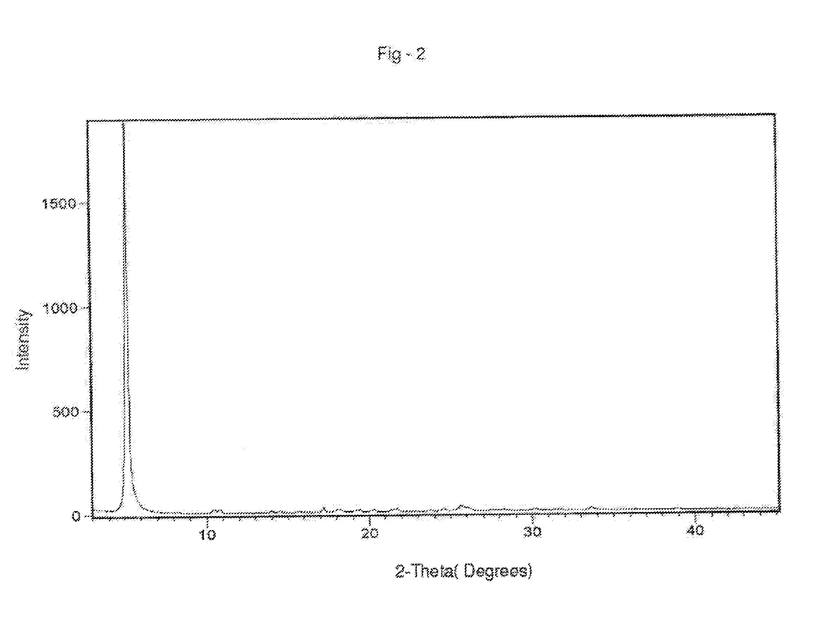

In another aspect, there is provided another crystalline form of ibandronate sodium designated as Form 11, characterized by X-ray powder diffraction pattern with characteristic peaks at about 5.2, 10.6, 17.2, 18.1, 21.6, 25.6, and 33.6 ± 0.2degrees two theta. Figure 2 shows a representative X-ray powder diffraction pattern for Form II.

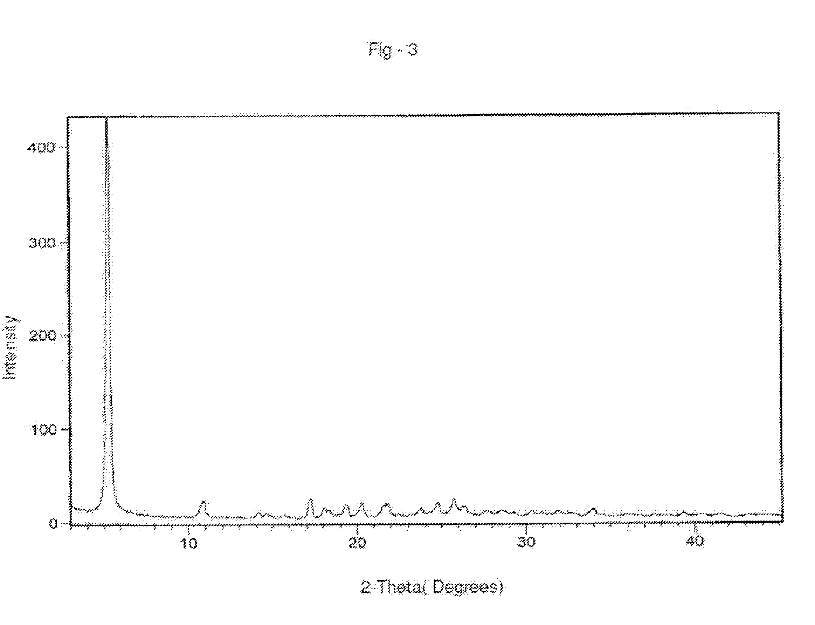

In another aspect, there is provided another crystalline form of ibandronate sodium designated as Form III, characterized by X-ray powder diffraction pattern with characteristic peaks approximately at: 5.4, 10.9, 14.4, 17.3, 18.2, 19.4, 20.3, 21.7, 24.7 and 25.7 ± 0.2 degrees two theta. Figure 3 shows a representative X-ray powder diffraction pattern for Form III.

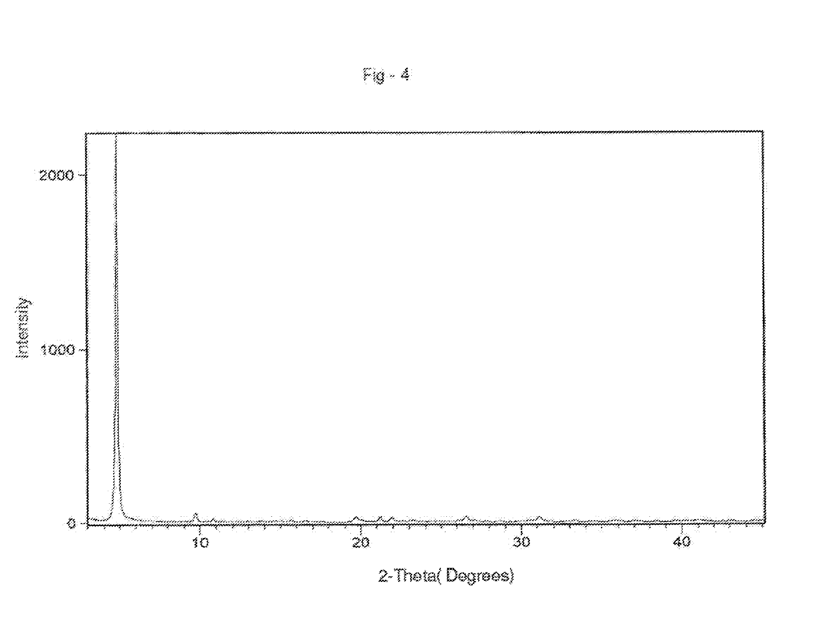

In another aspect, there is provided another crystalline form of ibandronate sodium designated as Form IV, characterized by X-ray powder diffraction pattern with characteristic peaks at about 4.9, 9.7, 19.7, 21.2, 21.9, 26.5, and 31.1 ± 0.2 degrees two theta. Figure 4 shows a representative X-ray powder diffraction pattern for Form IV.

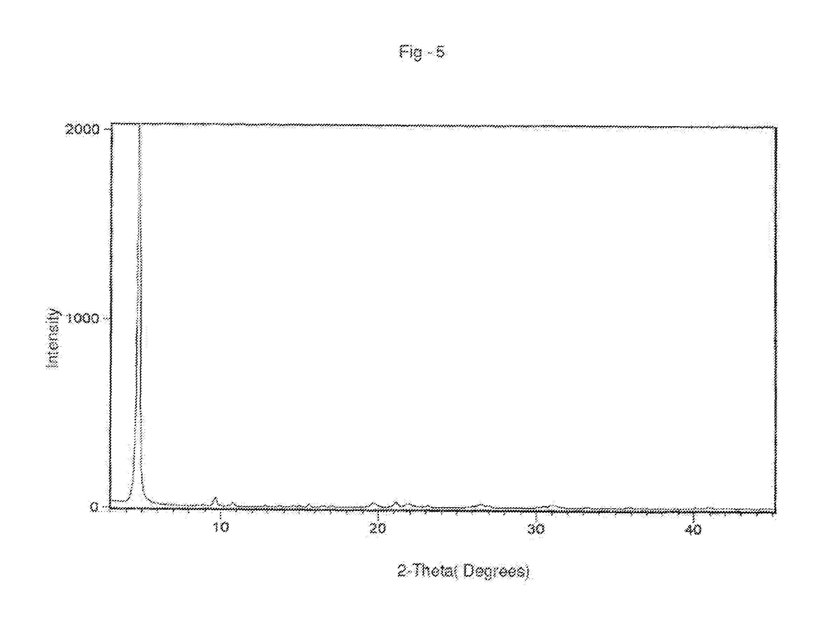

In another aspect, there is provided another crystalline form of ibandronate sodium designated as Form V, characterized by X-ray powder diffraction pattern with characteristic peaks at about 4.8, 10.8, 19.7, 22.0, and 31.1 ± 0.2degrees two theta. In addition to or instead of any of the characteristic peaks described herein above, the X-ray powder diffraction pattern may also include peaks at 9.7, 15.7, 20.0, 21.2, 26.4, 30.5, and 31.1 ±0.2 degrees 2 theta. Figure 5 shows a representative X-ray powder diffraction pattern for Form V.

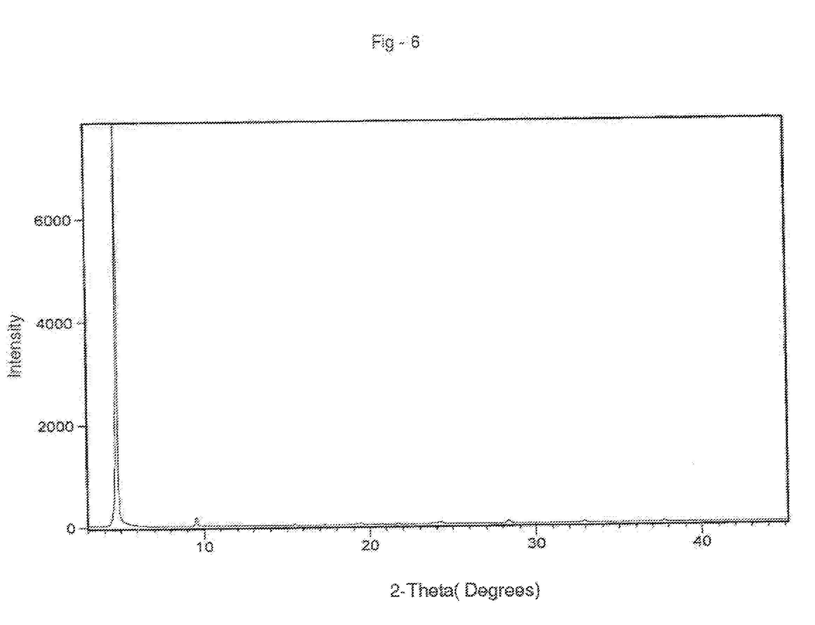

In another aspect, there is provided another crystalline form of ibandronate sodium designated as Form VI, characterized by X-ray powder diffraction pattern with characteristic peaks at about 4.8, 9.6, 15.4, 19.4, 21.7, 24.2, 28.2 32.9, and 37.7 ± 0.2 degrees two theta. Figure 6 shows a representative X-ray powder diffraction pattern for Form VI,

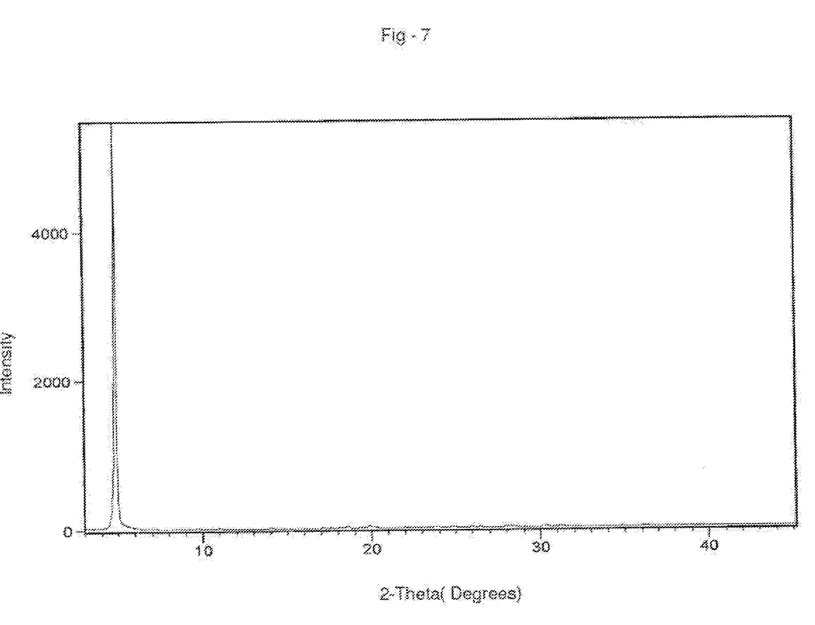

In another aspect, there is provided another crystalline form of ibandronate sodium designated as Form VII, characterized by X-ray powder diffraction pattern with characteristic peaks at about 4.9, 9.8, 10.9, 14.1, 17.1, 18.6, 19.8, 23.8, 24.8, and 25.9 ± 0.2 degrees two theta. Figure 7 shows a representative X-ray powder diffraction pattern for Form VII.

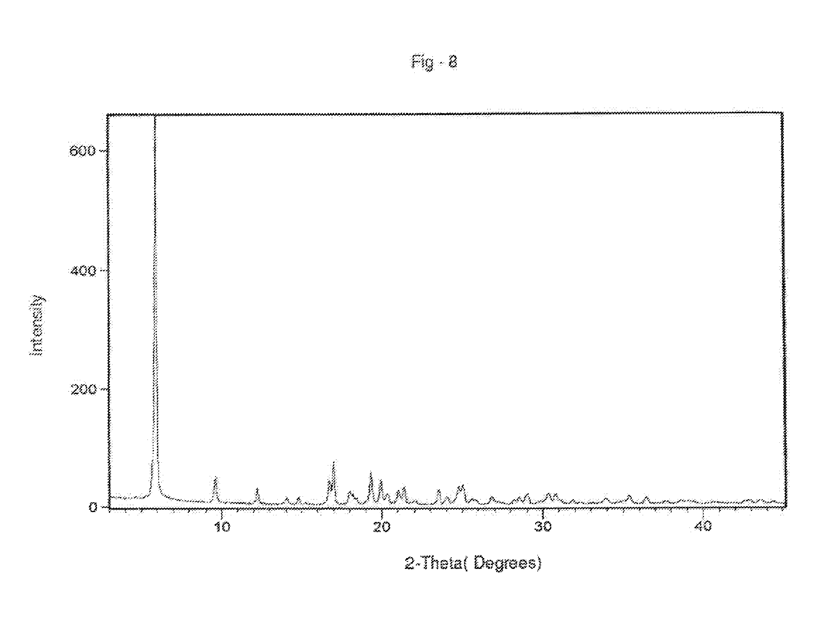

In another aspect, there is provided another crystalline form of ibandronate sodium designated as Form VIII, characterized by X-ray powder diffraction pattern with characteristic peaks at about 6.1, 16.7, 18.1, 20.3, and 30.2 ± 0.2 degrees two theta. In addition to or instead of any of the characteristic peaks described herein above, the X-ray powder diffraction pattern may also include peaks at 9.7, 17.1, 19.3, 20.0, 21.5, and 24.9 ±0.2 degrees. Figure 8 shows a representative X-ray powder diffraction pattern for Form VIII.

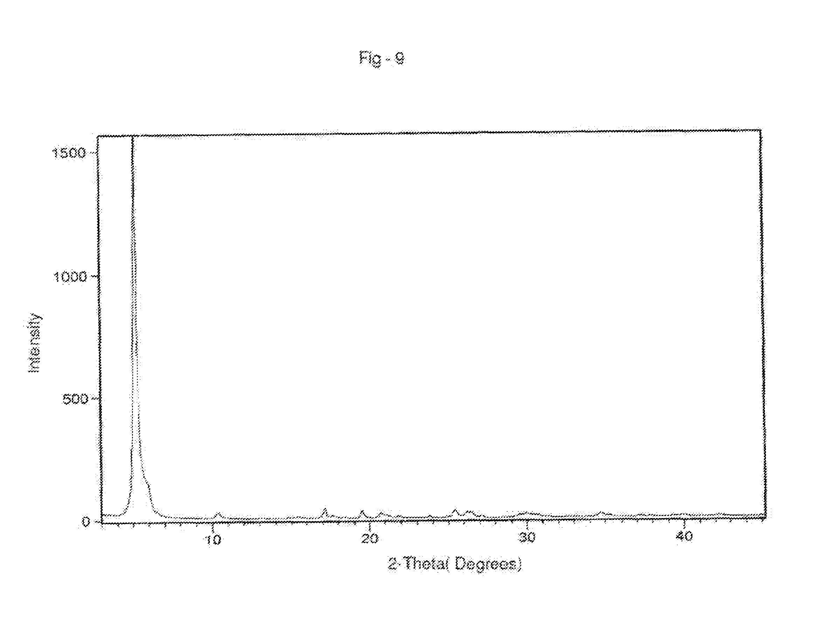

In another aspect, there is provided another crystalline form of ibandronate sodium designated as Form IX characterized by X-ray powder diffraction pattern with characteristic peaks at about 19.5, 20.8, 25.4, 26.3 and 34.9 ± 0.2 degrees two theta. Figure 9 shows a representative X-ray powder diffraction pattern for Form IX.

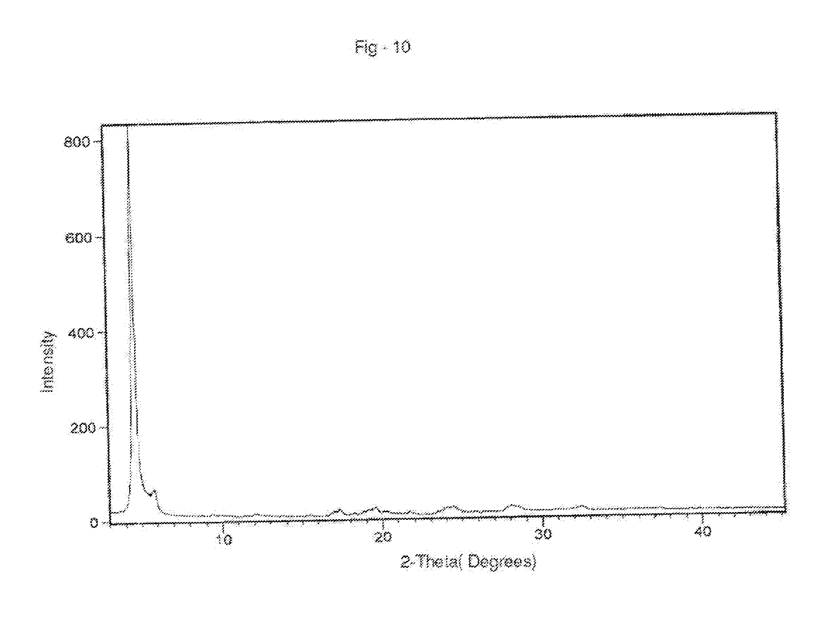

In another aspect, there is provided another crystalline form of ibandronate sodium designated as Form X characterized by X-ray powder diffraction pattern with characteristic peaks at about 4.6, 5.8, 17.2, 19.4, 24.4 and 28.0 ± 0.2 degrees two theta. Figure 10 shows a representative X-ray powder diffraction pattern for Form X.

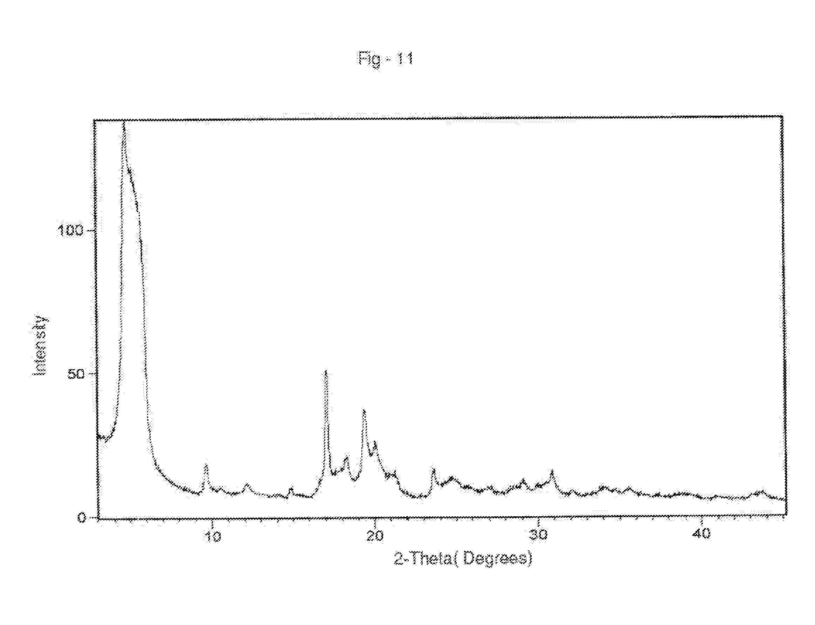

In another aspect, there is another crystalline form of crystalline ibandronate sodium designated as Form XI characterized by X-ray powder diffraction pattern with characteristic peaks at about 4.7, 5.9, 9.6, 17.1, and 19.4 ± 0.2 degrees two theta. Figure 11 shows a representative X-ray powder diffraction pattern for Form XI.

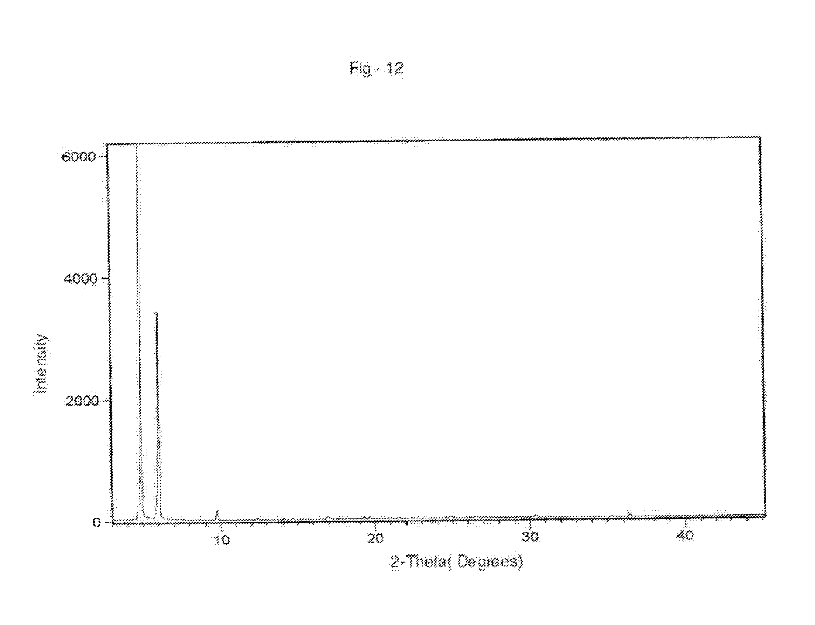

In another aspect, there is provided another crystalline form of ibandronate sodium designated as Form XII characterized by X-ray powder diffraction pattern with characteristic peaks at about 4.9, 6.0, 9.7, 12.4, 17.0, 19.3, 24.9, 29.3, 30.3 and 36.4 ± 0.2 degrees two theta. Figure 12 shows a representative X-ray powder diffraction pattern for Form XII.

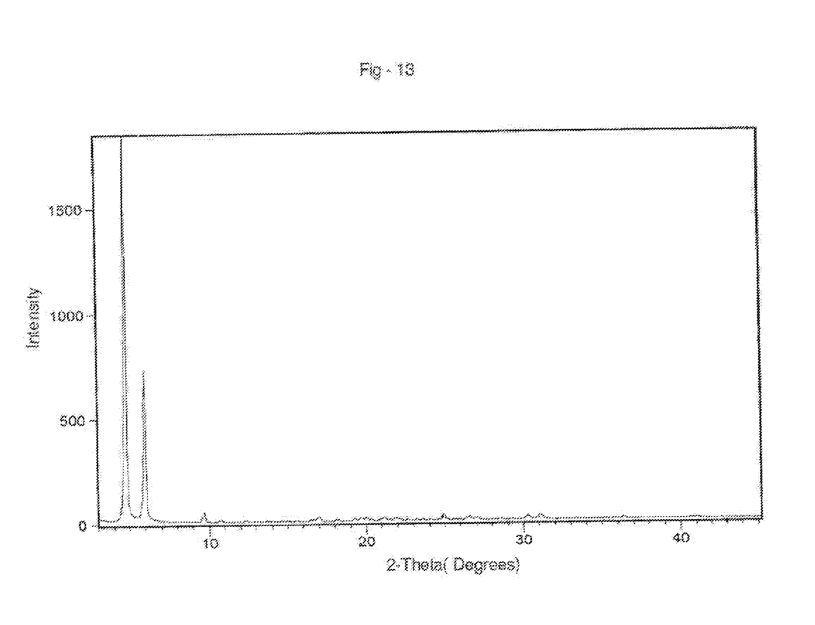

In another aspect, there is provided another crystalline form of ibandronate sodium designated as Form XIII characterized by X-ray powder diffraction pattern with characteristic peaks at about 4.8, 6.0, 9.7, 16.9, 19.7, 24.9 and 31.1 ± 0.2 degrees two theta. Figure 13 shows a representative X-ray powder diffraction pattern for Form XIII.

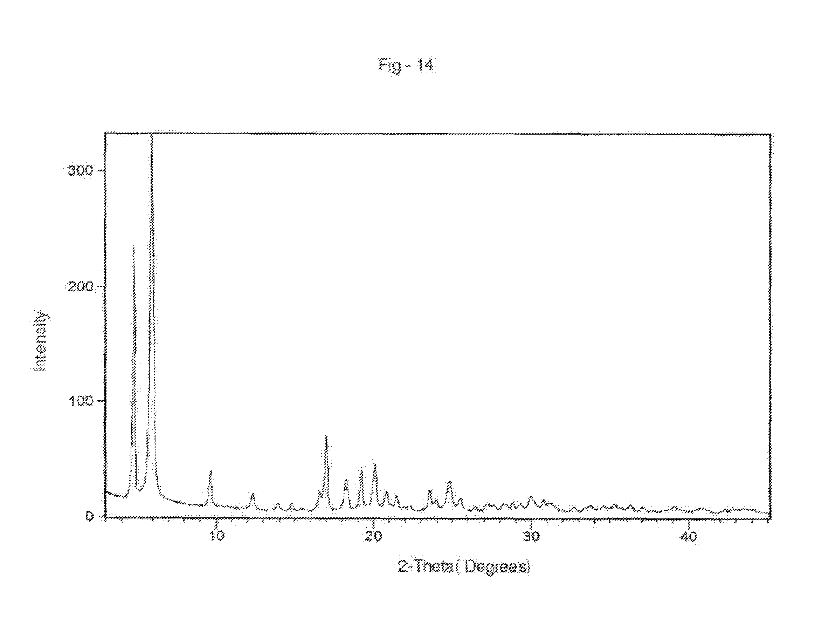

In another aspect, there is provided another crystalline form of ibandronate sodium designated as Form XIV characterized by X-ray powder diffraction pattern with characteristic peaks at about 4.8, 6.0, 9.6, 17.0, 18.2, 19.2, 20.1, and 24.8 + 0.2 degrees two theta. Figure 14 shows a representative X-ray powder diffraction pattern for Form XIV.

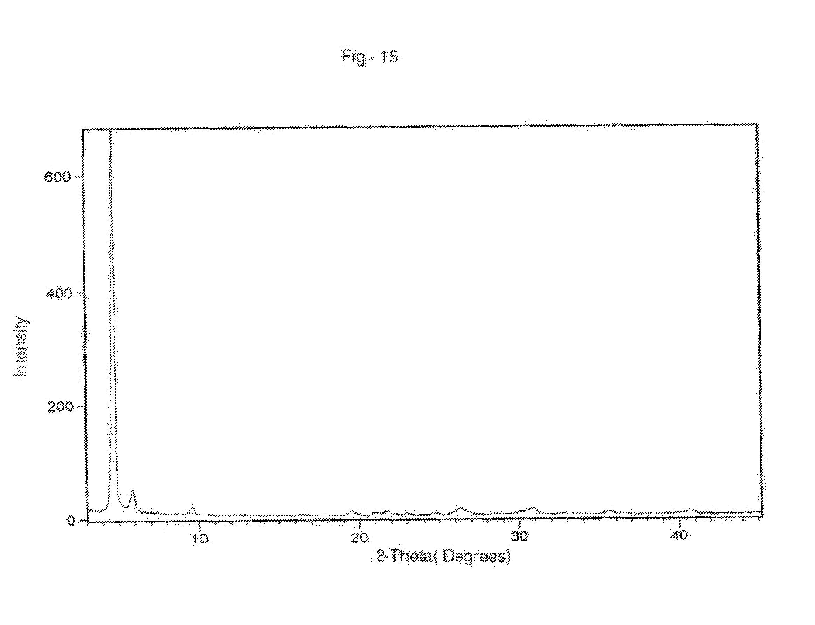

In another aspect, there is provided another crystalline form of ibandronate sodium designated as Form XV characterized by X-ray powder diffraction pattern with characteristic peaks at about 4.7, 5.9, 9.6, 19.5, 21.4, 26.3, 30.8, and 35.7 ± 0.2 degrees two theta. Figure 15 shows a representative X-ray powder Diffraction pattern for Form XV.

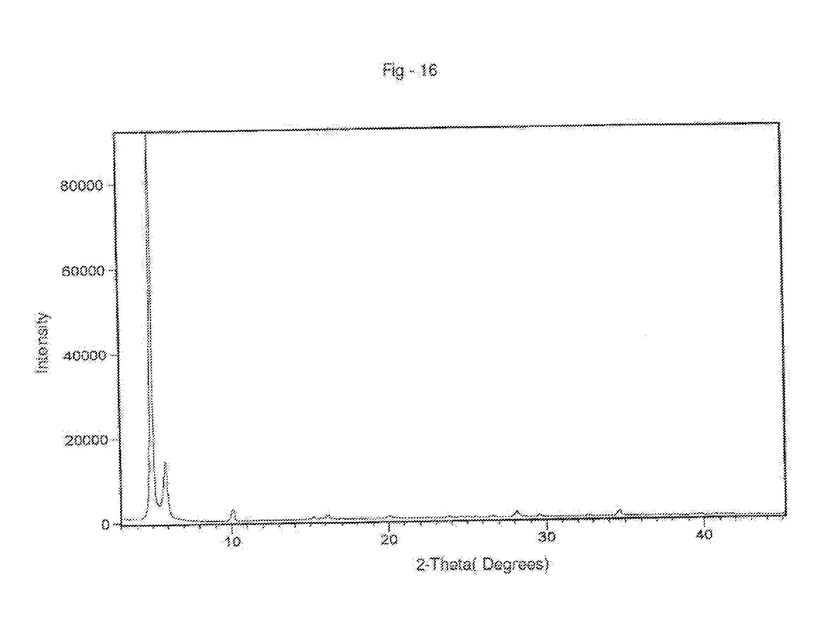

In another aspect, there is provided another crystalline form of ibandronate sodium designated as Form XVI characterized by X-ray powder diffraction pattern with characteristic peaks at about 5.0, 5.9, 10.1, 15.2, 15.7, 16.1, 17.0, 20.0, 22.4, and 23.8 ± 0.2 degrees two theta. Figure 16 shows a representative X-ray powder diffraction pattern for Form XVI.

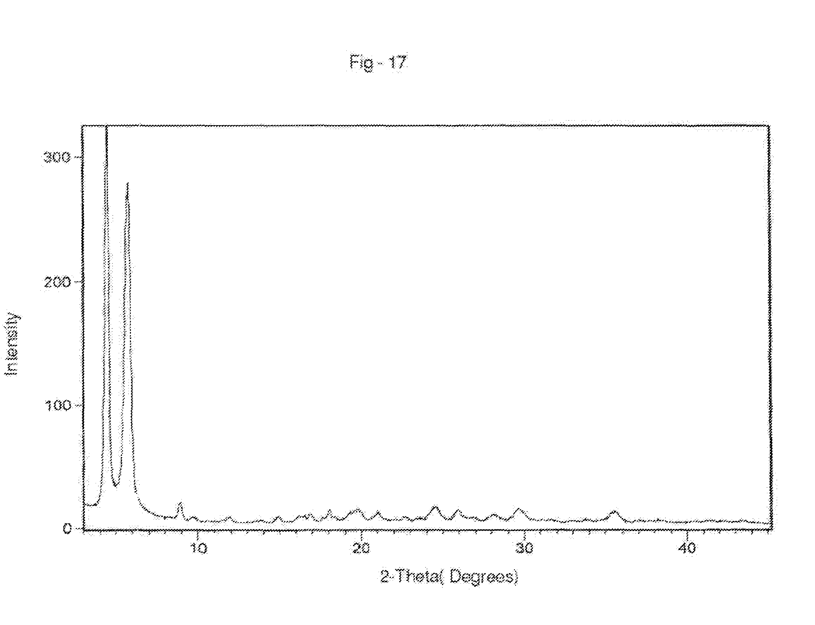

In another aspect, there is provided another crystalline form of ibandronate sodium designated as Form XVII characterized by X-ray powder diffraction pattern with characteristic peaks at about 4.5, 5.8, 8.9, 18.1, 19.8, 24.5, 25.9, 29.6 and 35.5 ± 0.2 degrees two theta. Figure 17 shows a representative X-ray powder diffraction pattern for Form XVII.

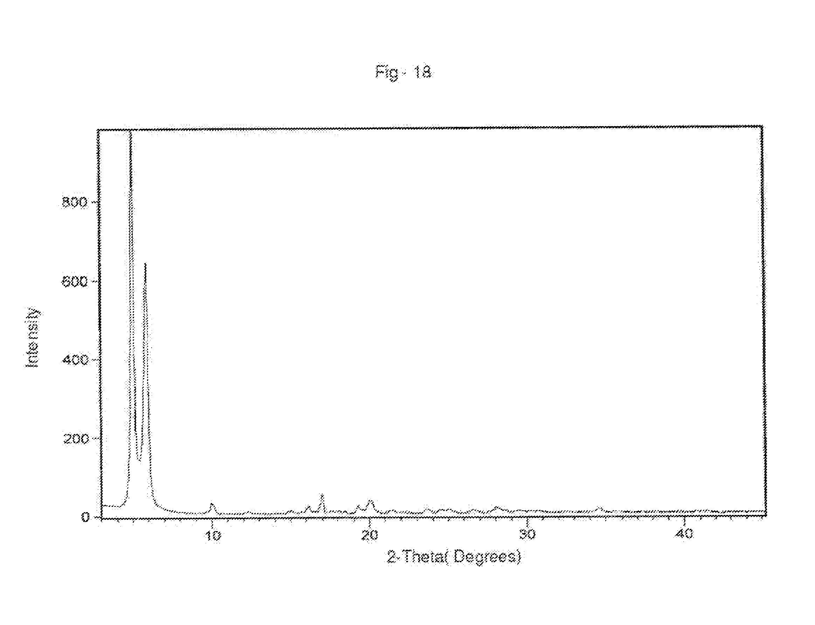

In another aspect, the present invention provides another crystalline form of ibandronate sodium designated as Form XVIII characterized by X-ray powder diffraction pattern with characteristic peaks at about 5.0, 5.9, 10.0, 17.0, 20.1, and 28.1 ± 0.2 degrees two theta. Figure 18 shows a representative X-ray powder diffraction pattern for Form XVIII.

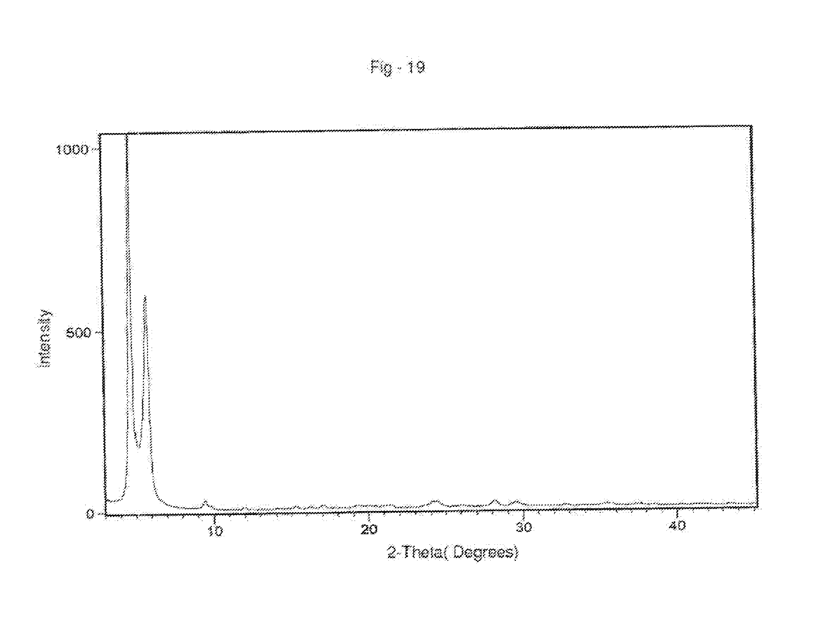

In another aspect, there is provided another crystalline form of ibandronate sodium designated as Form XIX characterized by X-ray powder diffraction pattern with characteristic peaks at 4.7, 5.7, 9.4, 17.0, 21.4, 24.3, 28.2, 29.5, and 35.4 ± 0.2 degrees two theta. Figure 19 shows a representative X-ray powder diffraction pattern for XIX.

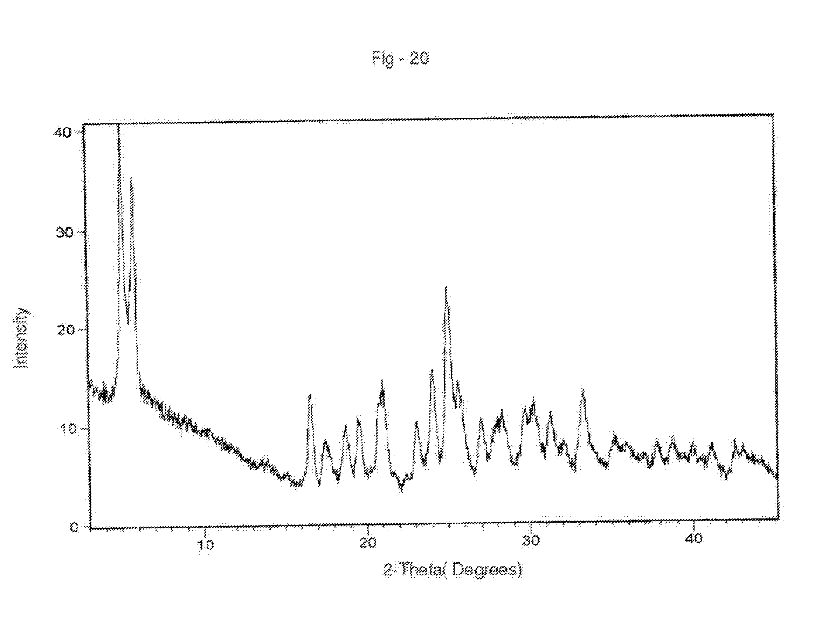

In another aspect, there is provided another crystalline form of ibandronate sodium designated as Form XX characterized by X-ray powder diffraction pattern with characteristic peaks at about 5.1, 5.8, 16.7, 20.8, 25.0, and 33.3 ±0.2 degrees two theta. Figure 20 shows a representative X-ray powder diffraction pattern for Form XX.

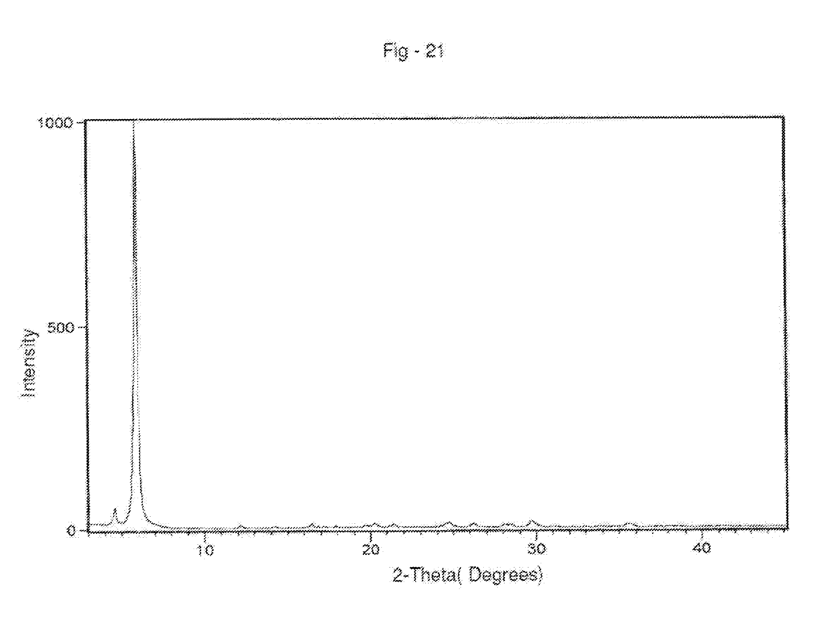

In another aspect, there is provided another crystalline form of ibandronate sodium designated as Form XXI characterized by X-ray powder diffraction pattern with characteristic peaks at about 4.6, 5.9, 16.4, 20.2, 24.7, 26.2, 28.2, 29.7 and 35.6 ± 0.2 degrees two theta. Figure 21 shows a representative X-ray powder diffraction pattern for Form XXI.

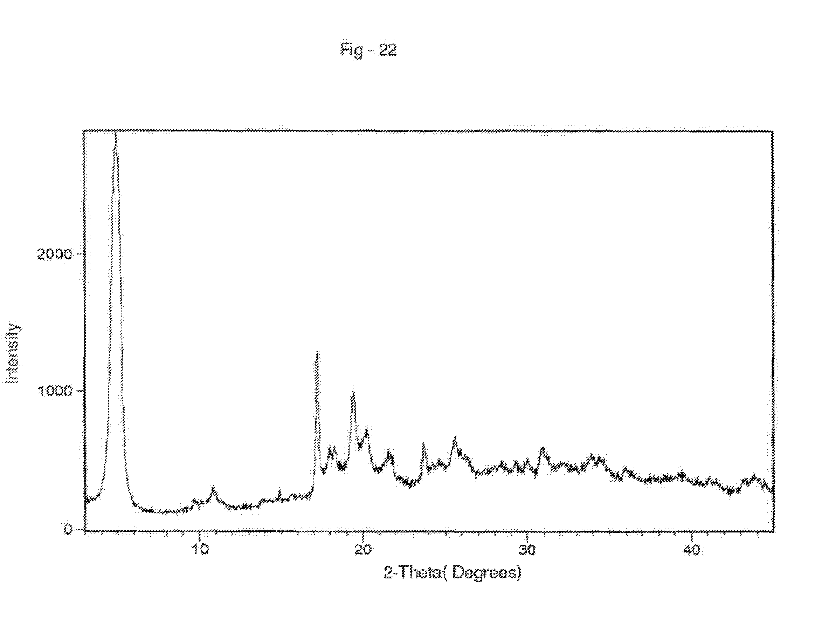

In another aspect, there is provided another crystalline form of ibandronate sodium designated as Form XXII characterized by X-ray powder diffraction pattern with characteristic peaks at about 5.2, 17.2, 19.4, 20.2, and 25.6 ± 0.2 degrees two theta. Figure 22 shows a representative X-ray powder diffraction pattern for Form XXII.

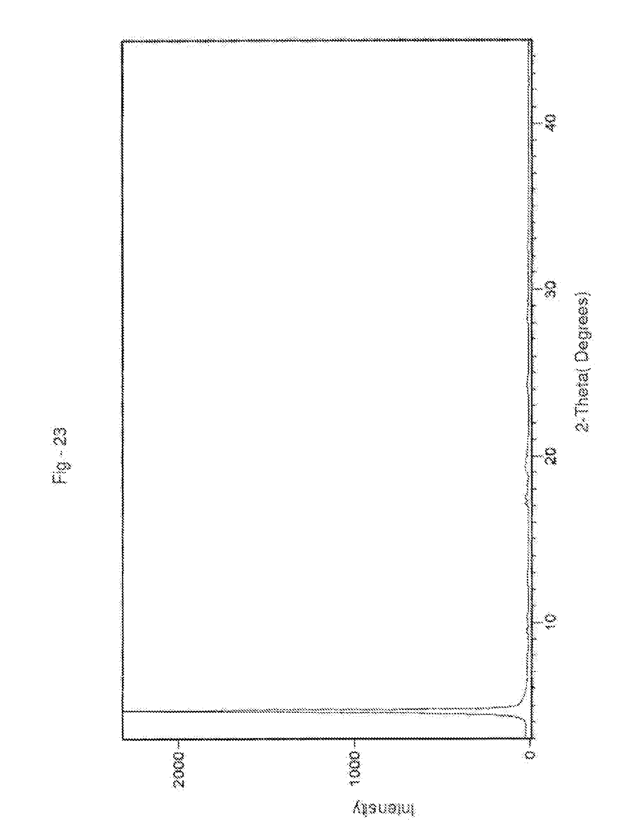

In another aspect, there is provided another crystalline form of ibandronate sodium designated as Form XXIII characterized by X-ray powder diffraction pattern with characteristic peaks at about 4.7, 12.7, 17.1, 17.5, 19.2, and 28.3 ± 0.2 degrees two theta. Figure 23 shows a representative X-ray powder diffraction pattern for Form XXIII.

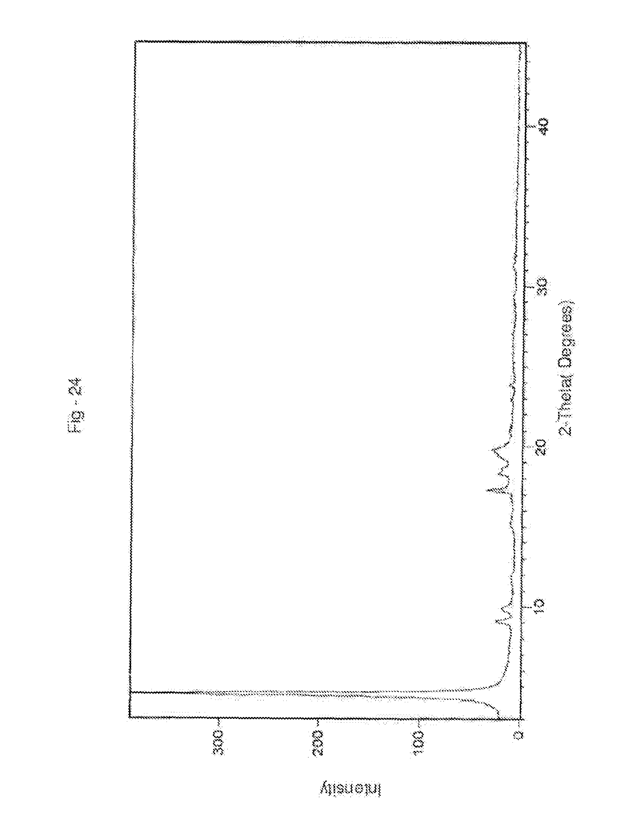

In another aspect, there is provided another crystalline form of ibandronate sodium designated as Form XXIV characterized by X-ray powder diffraction pattern with characteristic peaks at about 4.6, 9.1, 17.3, 18.5, and 19.7 ± 0.2 degrees two theta. Figure 24 shows a representative X-ray powder diffraction pattern for Form XXIV.

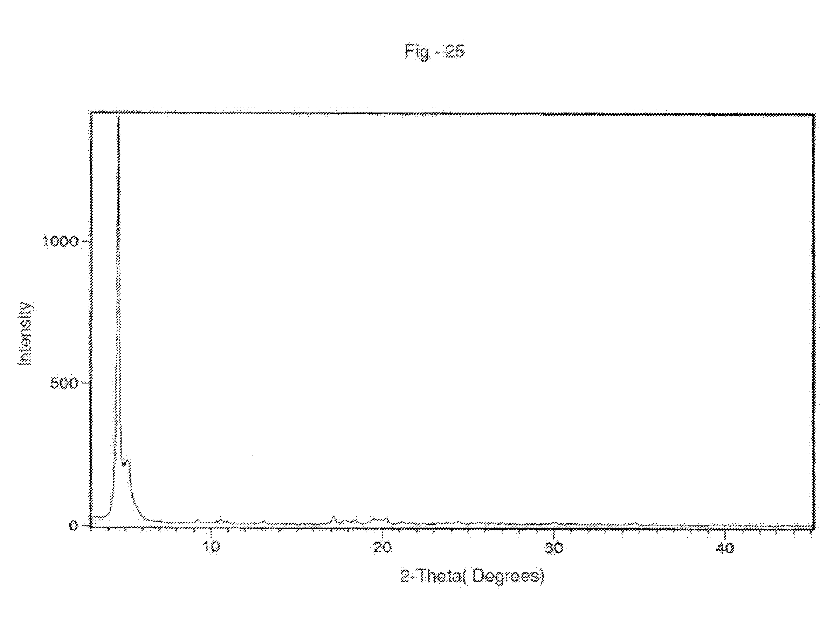

In another aspect, there is provided another crystalline form of ibandronate sodium designated as Form XXV characterized by X-ray powder diffraction pattern with characteristic peaks at about 4.6, 5.2, 10.6, 13.0, 17.1, 19.4, 20.2 and 34.6 ± 0.2 degrees two theta. Figure 25 shows a representative X-ray powder diffraction pattern for Form XXV.

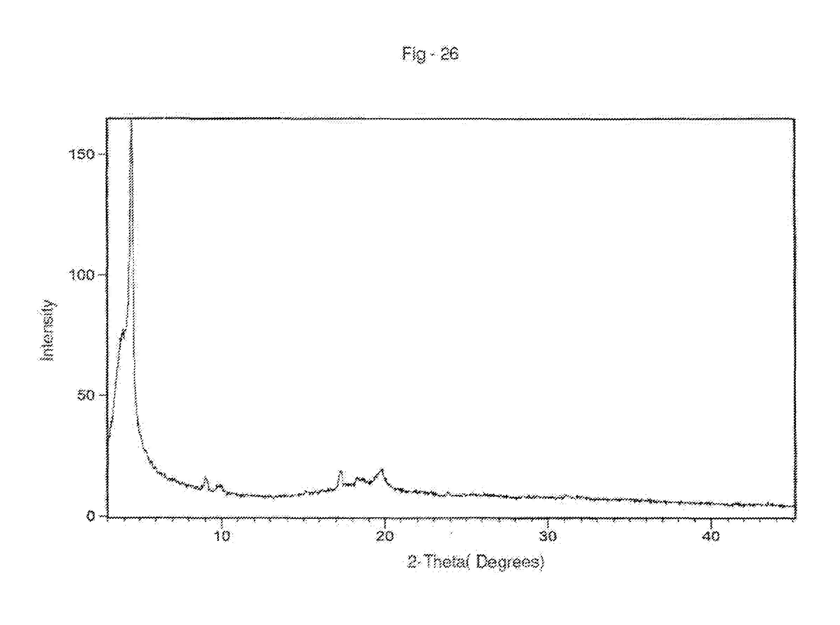

In another aspect, there is provided another crystalline form of ibandronate sodium designated as Form XXVI characterized by X-ray powder diffraction pattern with characteristic peaks at 3.8, 4.5, 9.0, 9.9, 17.3, 18.4 and 19.8 ± 0.2 degrees two theta. Figure 26 shows a representative X-ray powder diffraction pattern for Form XXVI.

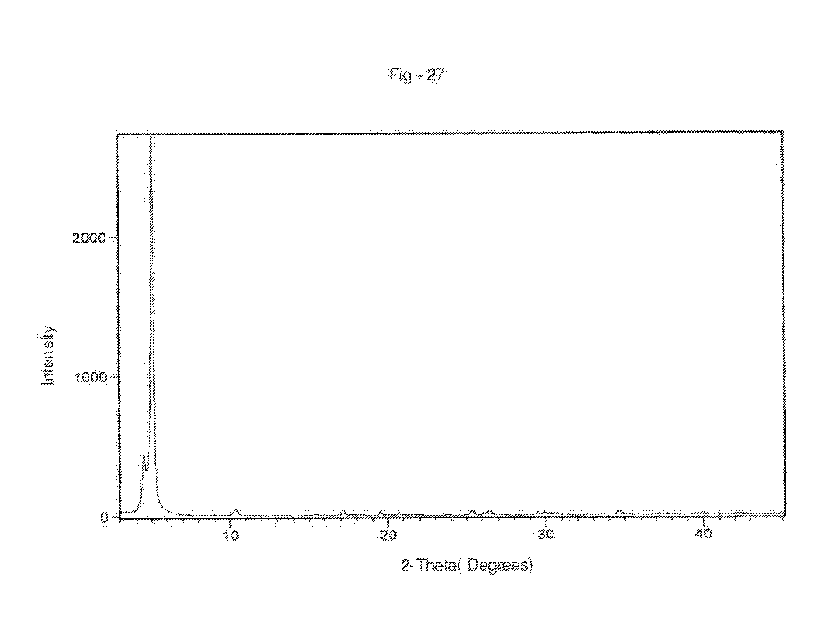

In another aspect, there is provided another crystalline form of ibandronate sodium designated as Form XXVII characterized by X-ray powder diffraction pattern with characteristic peaks approximately at: 4.5, 5.1, 10.3, 15.4, 17.1, 19.5, 20.7, 25.3, 26.4 and 30.6 ± 0.2 degrees two theta. Figure 27 shows a representative X-ray powder diffraction pattern for Form XXVII.

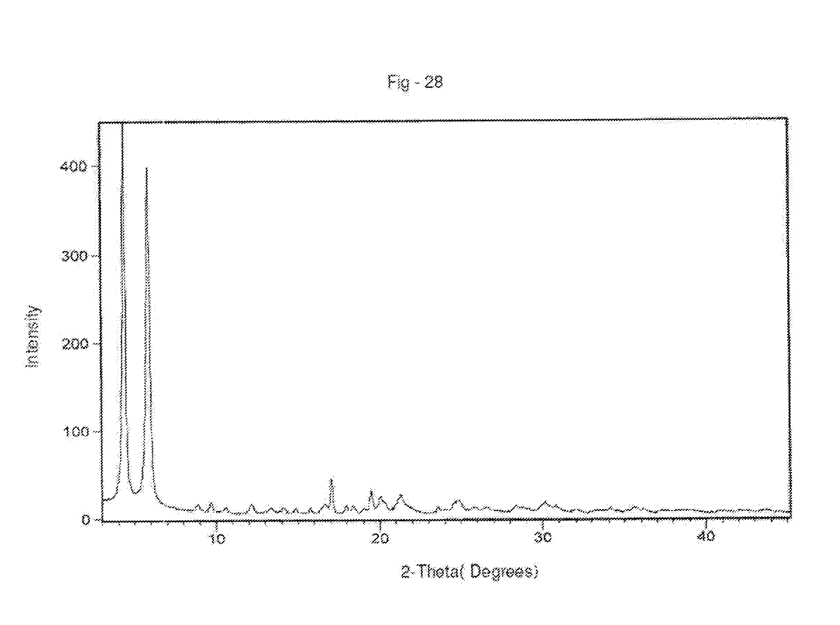

In another aspect, there is provided another crystalline form of ibandronate sodium designated as Form XXVIII characterized by X-ray powder diffraction pattern with characteristic peaks at 4.4, 5.9, 9.7, 12.1, 17.1, 19.4, 21.3, 24.9, 30.3 and 35.8 ± 0.2 degrees two theta. Figure 28 shows a representative X-ray powder diffraction pattern for Form XXVIII.

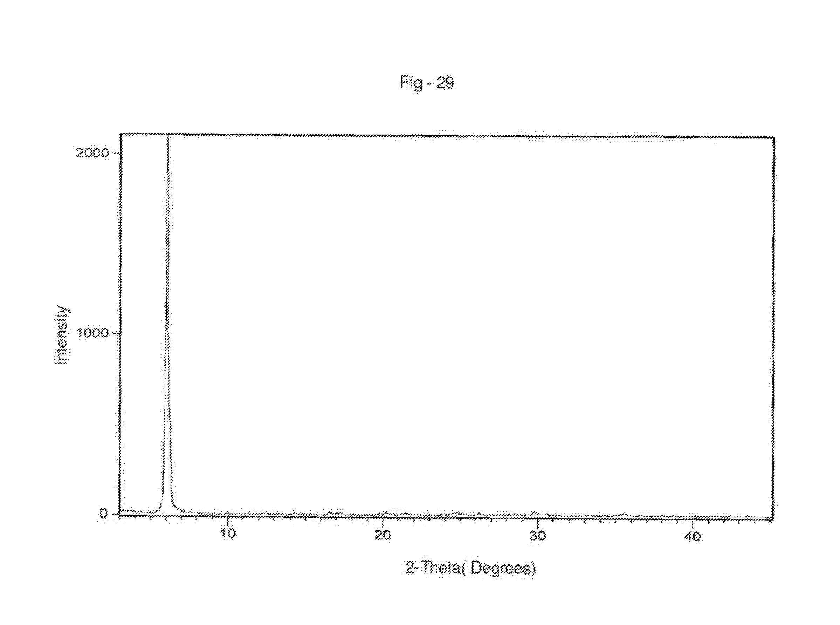

In another aspect, there is provided another crystalline form of ibandronate sodium designated as Form XXIX characterized by X-ray powder Diffraction pattern with characteristic peaks at 6.0, 6.3, 12.4, 14.3, 16.5, 19.7, 20.2, 21.5, 24.7, and 26.2 ± 0.2 degrees two theta. Figure 29 shows a representative X-ray powder diffraction pattern for Form XXIX.

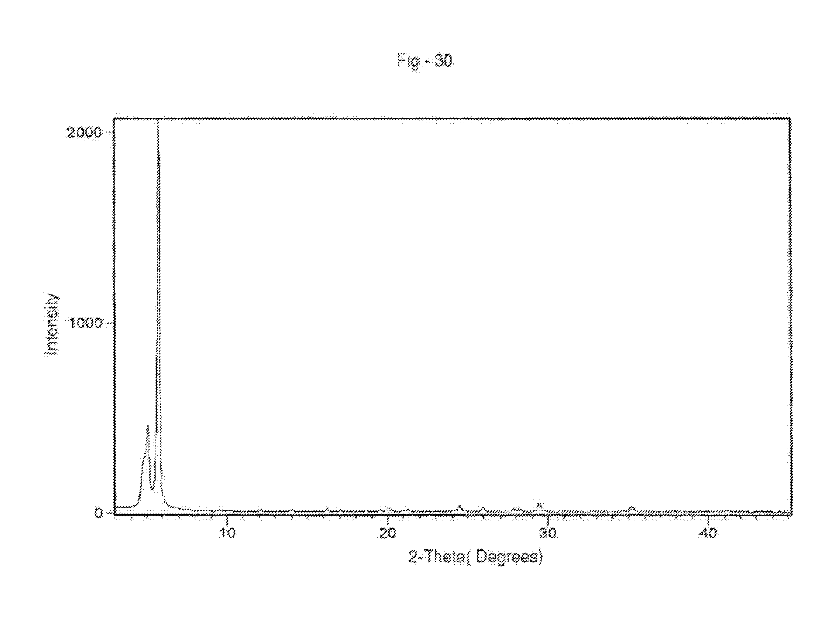

In another aspect, there is provided another crystalline form of ibandronate sodium designated as Form XXX characterized by X-ray powder diffraction pattern with characteristic peaks at about 4.7, 5.1, 5.7, 14.0, 16.2, 20.0, 21.1, 24.5, 25.9, and 28.0 ± 0.2 degrees two theta. Figure 30 shows a representative X-ray powder diffraction pattern for Form XXX.

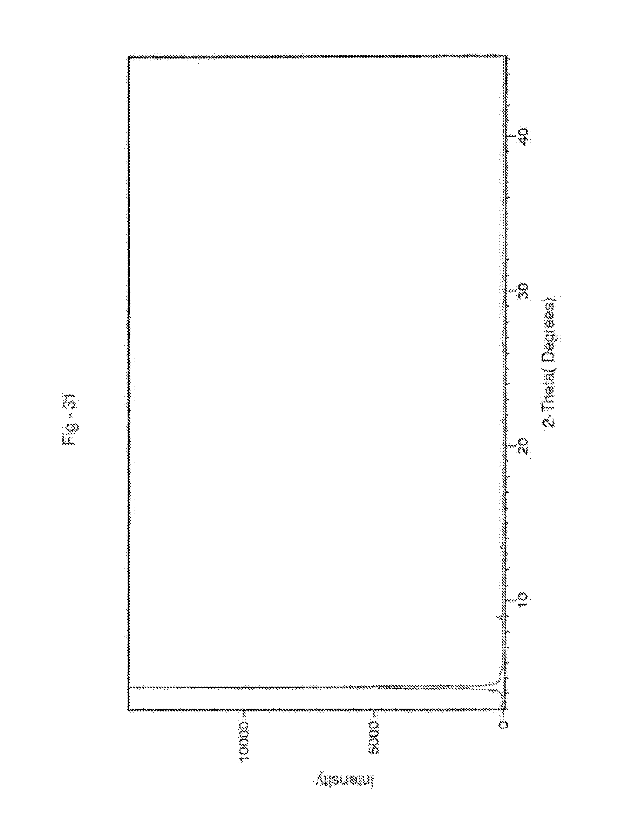

In another aspect, there is provided another crystalline form of ibandronate sodium designated as Form XXXI characterized by X-ray powder diffraction pattern with characteristic peaks at about 4.5, 4.7, 8.9, 13.4, and 26.6 ± 0.2 degrees two theta. Figure 31 shows a representative X-ray powder diffraction pattern for Form XXXI.

In yet another aspect, there is provided another crystalline form of ibandronate sodium designated as Form Alpha characterized by X-ray powder diffraction pattern with characteristic peaks at about 5.5, 6.3, 19.3, and 23,1 ± 0.2 degrees two theta. In addition to or instead of any of the characteristic peaks described herein above for Form Alpha, the X-ray powder diffraction pattern may also include peaks at 11.0, 15.0, 18.4, 21.0, 22.4, 28.1, and 33.3 ±0.2 degrees.

Figure 32 shows a representative X-ray powder diffraction pattern for Form Alpha.

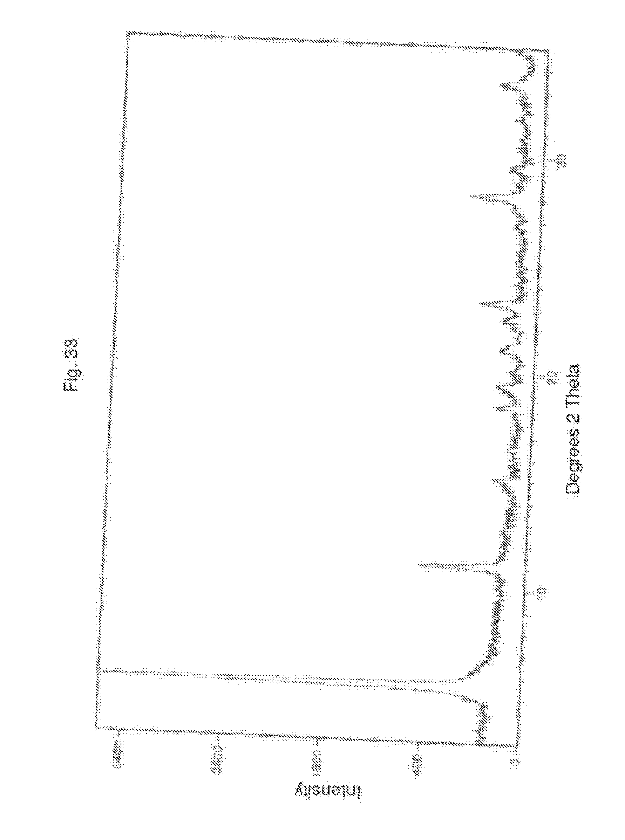

In yet further another aspect there is provided another crystalline form of ibandronate sodium designated as Form Beta characterized by X-ray powder diffraction pattern with characteristic peaks at about 5.5, 10.9, 19.3, 23.1, and 33.2 ±0.2 degrees two theta. In addition to or instead of any of the characteristic peaks described herein above, the X-ray powder diffraction pattern may also include peaks at 14.9, 18.3, 20.9, 22.4, 23.1, and 27.9 ±0.2 degrees. As mentioned above, it should be kept in mind that XRPD patterns for the same solid form typically vary as a function of a number of relevant factors, some of which include X-ray diffraction equipment and operator-to-operator variability. Figure 33 shows a representative X-ray powder diffraction pattern for Form Beta.

In further another aspect, there is provided a process for the preparation of ibandronate sodium crystalline Form-1, Form III, Form IV, Form V, Form VII, Form VIII, Form X, Form XI, Form XII, Form XIII, Form XIV, Form XV, XII, XIII, XIV, Form XXV, Form XXVI, Form XXVII, Form XXVIII, which comprises the steps of:
Step-1) providing a solution of ibandronate sodium in a suitable solvent or mixture of solvents;
Step-2) saturating the solution of step (1) by adding a suitable antisolvent or mixture of antisolvents;
Step-3) stirring the solution of step (2) at suitable temperature for a suitable time to precipitate the solid;
Step4) recovering the solid of step (3) by conventional methods to afford the desired crystalline Form of ibandronate sodium.
All the above four steps are individually described herein as disclosure of the present invention.

### Step 1) providing a solution of ibandronate sodium

The solution of ibandronate sodium may be obtained by dissolving ibandronate sodium in a suitable solvent, or such a solution may be obtained directly from a reaction in which ibandronate sodium is formed.

Suitable solvents which can be used for dissolution of ibandronate sodium include but are not limited to: water; acidic solvents such as formic acid, acetic acid and the like; formamide, DMSO; and mixtures thereof.

The temperature for dissolution can range from about 25°C to about 100°C.

The time period can be as long as required for the complete dissolution of ibandronate sodium, however may range from about 30 minutes to about 10 hours, or more.

### Step 2) Saturating the solution of step (1), by_adding a suitable antisolvent or mixture of antisolvents under suitable conditions

Saturating the solution of step (1) may be carried out by adding a suitable antisolvent or mixture of antisolvents under suitable conditions. The suitable antisolvents that can be used for saturation include but are not limited to: hydrocarbon solvents such as n-hexane, n-heptane, cyclohexane, and the like; ketones such as acetone, n-butanone, methyl isobutyl ketone and the like; nitriles such as acetonitrile, propionitrile and the like; alcoholic solvents such as methanol, ethanol, isopropyl alcohol, n-propanol, and the like; and ethers such as dimethyl ether, diethyl ether, diisopropyl ether, tetrahydrofuran, and the like. Mixtures of any of these solvents are also contemplates,

The temperature for saturation may range from about -20°C to about 35°C.

### Step 3) stirring the solution of step (2)

The solution of step (2) may be stirring at suitable temperature for a appropriate time to precipitate the solid material;

The suitable temperatures for carrying out the step (3) may range from about 1 10°C to about 35°C, preferably about 30°C.

The time period for stirring the solution may be in the range from about 30 minutes to about 10 hours, or longer hours, however it may extend as long as the complete precipitation of ibandronate sodium solid material is achieved.

### Step 4) recovering the solid of step (3)

Solid material obtained after step 3) may be recovered by conventional methods to afford the desired crystalline Form of ibandronate sodium.

The method by which the solid material is recovered from the final mixture, with or without cooling below the operating temperature, may be opted from any of the techniques such as filtration by gravity, or by suction, centrifugation, and the like. The crystals so isolated may carry a small proportion of occluded mother liquor containing a higher percentage of impurities. If desired the crystals may be washed on the filter with a solvent to wash out the mother liquor.

The wet cake obtained in step (4) may optionally be further dried.

Optional drying may be carried out in conventional equipments, which may be selected from tray dryer, vacuum oven, air oven, fluidized bed drier, spin flash dryer, flash dryer and the like. The drying temperatures for carrying out the drying of the crystalline polymorphic form may range from about 30°C to about 90°C with or without vacuum, depending upon the polymorphic form and its nature. The drying may be carried out for any desired time until the desired product quality parameters are achieved. Preferably, time periods may range from about 1 to 20 hours.

In one of the preferred embodiment of the process, the process involves dissolving ibandronate sodium in solvents like water, acetic acid, formic acid, or DMSO at a temperature ranging between about 50°C to about 160°C as per solvent utilized, followed by addition of ethylene glycol as an antisolvent at a temperature of about 30°C to affords crystalline Form I of ibandronate sodium.

In another embodiment of the process, the process involves dissolving ibandronate sodium in formamide at about 110°C to about 130°C followed by addition of antisolvent selected from ethanol, n-propanol, or n-butanol at a temperature of about 30°C to affords crystalline Form III of ibandronate sodium.

In another embodiment of the process, the process involves dissolving ibandronate sodium in formic acid at about 50°C to 70°C followed by addition of ethyl methyl ketone or n-propanol as antisolvent at about 50°C to afford crystalline Form IV of ibandronate sodium.

In another preferred embodiment of the process, the process involves dissolving ibandronate sodium in formic acid at a temperature about 50°C to about 65°C followed by addition of methyl tertiary butyl ether as antisolvent to afford crystalline Form V of ibandronate sodium. The crystalline form V is a stable solvatomorph of ibandronate sodium with formic acid having a DSC thermogram recorded peak at about 144.8°C, with onset at 133.3°C and end set at about 160°C.

In another embodiment of the process, the process involves dissolving ibandronate sodium in water at about 100°C followed by addition of isopropyl alcohol as antisolvent at about 30°C to afford crystalline Form VII of ibandronate sodium.

In another preferred embodiment of the process, the process involves dissolving ibandronate sodium in formic acid at about 50°C to about 60°C followed by addition of acetone as antisolvent to afford crystalline Form VIII of ibandronate sodium. Form VIII can be obtained by evaporating the solution of ibandronate sodium in water.

In another embodiment of the process, the process involves dissolving ibandronate sodium in water at about 100°C followed by addition of n-propanol as antisolvent and stirring at about 100°C to afford crystalline Form X of ibandronate sodium.

In another preferred embodiment of the process, the process involves dissolving ibandronate sodium in acetic acid at about 100°C followed by addition of isopropyl alcohol as antisolvent and stirring at about 70°C to afford crystalline Form XI of ibandronate sodium.

In another embodiment of the process, the process involves dissolving ibandronate sodium in formic acid at a temperature of about 60°C to 65°C followed by slow evaporation under ambient temperature condition to afford crystalline Form XII of ibandronate sodium.

In another embodiment of the process, the process involves dissolving ibandronate sodium in formic acid at a temperature of about 50°C to about 60°C followed by addition of acetonitrile as antisolvent followed by stirring at about 30°C affords crystalline Form XIII of ibandronate sodium.

In another embodiment of the process, the process involves dissolving ibandronate sodium in formic acid at about 60°C followed by addition of 1,4 dioxane as antisolvent and stirring at about 30°C affords crystalline Form XIV of ibandronate sodium.

In another embodiment of the process, the process involves dissolving ibandronate sodium in formic acid at about 60°C followed by addition of dichloromethane as antisolvent and stirring at about 30°C affords crystalline Form XV of ibandronate sodium.

In another embodiment of the process, the process involves dissolving ibandronate sodium in formamide at about 150 to 165°C followed by addition of n-butanol as antisolvent at 25 to 35°C and stirring for about 15 to 30 minutes to afford crystalline Form XXII of ibandronate sodium.

In another preferred embodiment of the process, the process involves dissolving ibandronate sodium in formamide at about 50 to 60°C followed by addition of n-propanol as antisolvent at about 50 to 60°C affords crystalline Form XXIII of ibandronate sodium.

In another preferred embodiment of the process, the process involves dissolving ibandronate sodium in DMSO at about 150 to 160°C followed by addition of ethyl acetate as antisolvent and stirring at about 30°C affords crystalline Form XXIV of ibandronate sodium.

In another preferred embodiment of the process, the process involves dissolving ibandronate sodium in formamide at about 120°C followed by addition of n-butanol as antisolvent and stirring at about 30°C affords crystalline Form XXV of ibandronate sodium.

In another embodiment of the process, the process involves dissolving ibandronate sodium in dimethyl sulfoxide (DMSO) at about 150°C followed by addition of methyl tertiary butyl ether as antisolvent and stirring at about 30°C affords crystalline Form XXVI of ibandronate sodium.

In another embodiment of the process, the process involves dissolving ibandronate sodium in water at about 100°C followed by addition of tetrahydrofuran (THF) as antisolvent and stirring at about 30°C affords crystalline Form XXVII of ibandronate sodium.

In another embodiment of the process, the process involves dissolving ibandronate sodium in acetic acid at about 60-65°C followed by addition of ethyl methyl ketone as antisolvent and stirring at about 30°C affords crystalline Form XXVIII of ibandronate sodium.

In yet another aspect, there is provided a process for the preparation of crystalline Form II, Form VI, Form XVI, Form XVII, Form XVIII, Form XIX, Form XX, Form XXI, Form XXIX, Form XXX of ibandronate sodium, which comprises the steps:
Step-01) suspending ibandronate sodium in a suitable solvent or mixture of solvents;
Step-02) stirring the suspension of Step 01);
Step-03) recovering the solid of Step 02) by convention methods to afford the desired crystalline Form of ibandronate sodium.
All the above three steps are individually described herein as disclosure of the present invention.

### Step-01) Suspending ibandronate sodium in a suitable solvent or mixture of solvents.

The process of suspending the ibandronate sodium in solvent or a mixture of solvent comprises mixing of the components. Suitable solvents, which may be used for suspending ibandronate sodium include but are not limited to formamide, N,N-dimethyl acetamide, Dimethyl formamide (DMF), water, formic acid, acetic acid, and the like; or mixtures thereof,

### Step-02) Stirring the suspension of step (1)

The temperatures for stirring the suspension may range from about -10°C to about 35°C.

The time period may be as long as required for the complete precipitation of ibandronate sodium, times from about 30 minutes to about 10 hours, or longer depending upon achieving the complete precipitation.

### Step 3) recovering the solid of step (2)

The solid may be recovered from the precipitate obtained in the step -02) by conventional methods known to the person skilled in the art, to afford the desired crystalline Form of ibandronate sodium.

Conventional techniques/methods utilized for the recovery of solid crystalline form may be selected from filtration, decantation, centrifugation and the like in the presence or absence of inert atmosphere such as for example nitrogen and the like.

The wet cake obtained in step (3) may be optionally further dried, wherever, required, depending upon the desired characteristics of the crystalline polymorphic form.

Drying may be suitably carried out in a equipment selected from tray dryer, vacuum oven, air oven, fluidized bed drier, spin flash dryer, flash dryer and the like. The drying can be carried out at temperatures of about 30°C to about 90°C with or without vacuum. The drying can be carried out for any desired time until the required product purity is achieved, time periods from about 1 to 20 hours frequently being sufficient.

In one preferred embodiment of the above process using formamide as solvent and stirring at about 0-5°C or 50-60°C for about 10 hours affords ibandronate sodium crystalline Form II.

In one preferred embodiment of the above process, by using dimethylformamide as solvent and stirring at about 50 to about 60°C for about 10 to 12 hours, to afford ibandronate sodium crystalline Form VI.

In one preferred embodiment of the above process, by using dimethylacetamide as solvent and stirring at about 50 to about 60°C for about 10 to 12 hours, affords ibandronate sodium crystalline Form XVI.

In one preferred embodiment of the above process, by using dimethylsulfoxide (DMSO) as solvent and stirring at about 25 to 35°C for about 10 hours, affords ibandronate sodium crystalline Form XVII.

In one preferred embodiment of the above process, by using dimethylacetamide as solvent and stirring at about 25 to 35°C for about 10 to 12 hours, affords ibandronate sodium crystalline Form XVIII.

In one preferred embodiment of the above process, by using dimethylformamide as solvent and stirring at about 25 to 35°C for about 10 to 12 hours, affords ibandronate sodium crystalline Form XIX.

In one preferred embodiment of the above process, by using water as solvent and stirring at about 0 to 5°C for about 10 to 12 hours, affords ibandronate sodium crystalline Form XX.

In one preferred embodiment of the above process, by using dimethyl sulfoxide as solvent and stirring at about 50-60°C for 10 to 12 hours, affords ibandronate sodium crystalline Form XXI.

In one preferred embodiment of the above process, by using formic acid as solvent and stirring at about 50-60°C for 10 to 12 hours, affords ibandronate sodium crystalline Form XXIX.

In one preferred embodiment of the above process, by using acetic acid as solvent and stirring at about 50 to 60°C for 10 hours, affords ibandronate sodium crystalline Form XXX.

In yet another embodiment of the present invention there is provided a process for the preparation of crystalline Form IX of ibandronate sodium, which comprises the steps:
Step 1) providing a solution of ibandronate sodium in a suitable solvent or mixture of solvents;
Step 2) recovery of the solid from step (1) to afford the desired crystalline Form IX of ibandronate sodium.
The above two steps are individually described herein as disclosure of the present invention.

### Step 1) providing a solution of ibandronate sodium in a suitable solvent or mixture of solvents:

Providing a solution of ibandronate sodium in a suitable solvent or mixture of solvents comprises dissolving the ibandronate sodium in a solvent or mixture of solvents at a suitable temperature. Suitable solvents that can be used for dissolution of ibandronate sodium include but are not limited to: water; alcoholic solvents such as methanol, ethanol, isopropyl alcohol, n-propanol, and the like; acidic solvents such as formic acid, acetic acid and the like; or mixtures thereof, Preferably, water is utilized for preparing solution.

The temperature for dissolution may range from about 25°C to about 100°C or reflux temperature of the solvents used.

### Step 2) recovery of the solid from step (1)

The solid crystalline form may be recovered by lyophilization or freeze drying of the solution obtained from the step 1) to afford the desired crystalline Form IX of ibandronate sodium.

In yet still further aspect, there is provided a process for the preparation of crystalline Form XXXI of ibandronate sodium, which comprises the steps:
Step-1) providing a solution of ibandronate sodium in suitable weak acid under suitable conditions;
Step-2) recovering the solid of step (1) by suitable conventional techniques to afford the desired crystalline Form of ibandronate sodium.

In the above process using acetic acid as solvent followed by stirring at about 60-65°C and keeping aside for slow evaporation of solvent affords crystalline Form XXXI of ibandronate sodium. Form XXXI may also be formed by evaporating the solution of ibandronate sodium in acetic acid slowly under ambient temperature.

In yet another aspect, there is provided a process for the preparation of novel crystalline Form designated as Form Alpha, which includes desolvation of ibandronate sodium formic acid solvate (Form V) until the formic acid content is less than about 1% w/w. The preferred mode of desolvation is by drying.

Crystalline Form Alpha can also be obtained by desolvation of any solvated polymorphic form of ibandronate sodium include but are not limited to formic acid solvate, acetic acid solvate, ethylene glycol solvate, DMSO solvate until the solvate content is less than about 1% w/w.

The crystalline ibandronate sodium Form Alpha of the present invention may have water content in the range of from about 0.1% to about 10 % w/w by Karl Fisher method.

The ibandronate sodium formic acid solvate (Form V) may be subjected to drying at a temperature of about 50°C to about 150°C, preferably at about 100°C. The drying may be carried out using direct contact dryers such as fluid bed dryer or indirect contact dryers such as tray dryer. The drying may be carried out with or without applying the vacuum. The exact time required for desolvation may be readily determined by a person skilled in the art. The same times and temperatures may be used for desolation of other solvates as described herein, to produce Form Alpha.

In another aspect, there is provided a process for preparing Crystalline form beta of ibandronate sodium, which comprises exposing Form Alpha to humid environment having moisture preferably more than 30%. Preferably a humid environment containing moisture content in the range from about 40% to about 80% is utilized.

Crystalline Form Beta can also be obtained by exposing any polymorphic form of ibandronate sodium of the present invention to humid environment.

The solid of crystalline ibandronate sodium Form Beta of the present invention may have water content in the range of about 0.1% to about 10 % w/w (by Karl Fisher method) and preferably has formic acid content of less than about 1 %w/w.

The humid environment may also be conducted in inert atmosphere in the presence of gases such as nitrogen, argon etc. The relative humidity of the humid environment to which the substrate form is exposed in the process is preferably more than 30%, more preferably in the range of about 40 - 80 %. The Form Alpha may be exposed to humidity at a temperature of about 20°C to about 80°C, preferably at about 50°C. The duration of exposure to humid atmosphere depends on the time required for conversion of Form Alpha to Form Beta, which in turn depends on parameters such as temperature and humidity of the atmosphere and can be readily determined by a person skilled in the art. The Form Alpha can also be exposed to humid atmosphere under controlled conditions by using humid air in suitable equipment such as fluid bed dryer or humidification chamber.

Exposing to humid environment also includes other methods such as exposing to atmospheric moisture at room temperature.

The starting material for the polymorphs of present invention can be crude or pure ibandronate sodium obtained by any method known in the art. The starting material for either process can also be in any polymorphic form, such as amorphous or crystalline forms or a mixture of amorphous and crystalline forms obtained by any method known in the art.

The starting material for preparing the polymorphs of present invention including Form Alpha and Form Beta may be crude or pure ibandronate sodium obtained by any method known in the art or of the present invention. The starting material for either process can also be in any polymorphic form, such as amorphous or crystalline forms of ibandronate sodium or a mixture of amorphous and crystalline forms of ibandronate sodium obtained by any method known in the art or of the present forms.

Optionally seeding crystals of individual crystalline Forms of ibandronate sodium can also be used in the process for the preparation crystalline Forms of ibandronate sodium of present invention.

All X-ray powder diffraction pattern data provided herein, were obtained using a PANalytical Axe D8 Advance Powder X-ray Diffractometer. XRPD pattern was recorded at wavelength 1.5418 A using Cu K α radiation.

The dried product can optionally be milled to get a desired particle size.

Milling or micronization can be performed prior to drying, or after the completion of drying of the product. The milling operation reduces the size of particles and increases surface area of particles by colliding particles with each other at high velocities.

Drying is more efficient when the particle sizes of the material are smaller and the surface area is higher, hence milling can be performed prior to the drying operation.

Milling can be done suitably using jet milling equipment like an air jet mill, or using other conventional milling equipment.

In yet another aspect, the present invention encompasses pharmaceutical compositions comprising crystalline polymorphs of ibandronate sodium of the present invention and at least one pharmaceutically acceptable carrier.

Polymorphs of ibandronate sodium of the present invention can be formulated as solid compositions for oral administration in the form of capsules, tablets, pills, powders or granules. In these compositions, the active product according to the invention is mixed with one or more pharmaceutically acceptable excipients. The active can be present in each dosage form in an amount from about 0.1 micrograms up to 2 grams. More typically, each dosage form will contain between about 1 mg and about 250 mg, more preferably, about 2.5 mg and about 150 mg. The drug substance can be formulated as liquid compositions for oral administration including for example solutions, suspensions, syrups, elixirs and emulsions, containing solvents or vehicles such as water, sorbitol, glycerine, propylene glycol or liquid paraffin, may be used.

The compositions for parenteral administration can be suspensions, emulsions or aqueous or non-aqueous, sterile solutions. As a solvent or vehicle, propylene glycol, polyethylene glycol, vegetable oils, especially olive oil, and injectable organic esters, e.g. ethyl oleate, may be employed. These compositions can contain adjuvants, especially wetting, emulsifying and dispersing agents. The sterilization may be carried out in several ways, e.g. using a bacteriological filter, by incorporating sterilizing agents in the composition, by irradiation or by heating. They may be prepared in the form of sterile compositions, which can be dissolved at the time of use in sterile water or any other sterile injectable medium.

Pharmaceutically acceptable carriers that are of use in the present invention include but are not limited to diluents such as starch, pregelatinized starch, lactose, powdered cellulose, microcrystalline cellulose, dicalcium phosphate, tricalcium phosphate, mannitol, sorbitol, sugar and the like; binders such as acacia, guar gum, tragacanth, gelatin, polyvinyl pyrrolidone, hydroxypropyl cellulose, hydroxypropyl methyl cellulose, pregelatinized starch and the like; disintegrants such as starch, sodium starch glycolate, pregelatinized starch, crospovidone, croscarmellose sodium, colloidal silicon dioxide and the like; lubricants such as stearic acid, magnesium stearate, zinc stearate and the like; glidants such as colloidal silicon dioxide and the like; solubility or wetting enhancers such as anionic or cationic or neutral surfactants, complex forming agents such as various grades of cyclodextrins, resins; release rate controlling agents such as hydroxypropyl cellulose, hydroxymethyl cellulose, hydroxypropyl methyl cellulose, ethyl cellulose, methyl cellulose, various grades of methyl methacrylates, waxes and the like. Other pharmaceutically acceptable excipients that are of use include but not limited to film formers, plasticizers, colorants, flavoring agents, sweeteners, viscosity enhancers, preservatives, antioxidants and the like.

The methods used for differential scanning calorimetric (DSC) analysis were determined in a DSC Q200 V9.4 Build 287 model from TA Instruments with a ramp of 10°C/min to 250°C. For thermal gravametric analysis (TGA) curve generation, a TGA Q500 V6.4 Build 193 instrument was used with a ramp of 10°C/min up to 380°C

Having described the invention with reference to certain specific aspects and embodiments, other embodiments will become apparent to one skilled in the art from consideration of the specification. The invention is further defined by reference to the following examples describing in greater detail certain specific aspects and embodiments, the examples not being intended to limit the scope of the invention in any manner. It will be apparent to those skilled in the art that many modifications, both to materials and methods, may be practiced without departing from the scope of the invention.

### EXAMPLES

### EXAMPLE 1: PREPARATION OF IBANDRONATE SODIUM CRYSTALLINE FORM I

Ibandronate sodium (800 mg) was charged into a clean and dry round 4neck bottom flask containing water (3 ml). The mixture heated to 100°C and stirred for 15 minutes. The resultant homogenous reaction solution was cooled to room temperature and ethylene glycol (15 ml) was charged in one portion. The resultant reaction mixture was stirred at room temperature for 10 to 15 minute. The separated solid was filtered and subjected to suction pressure for 30 minutes to afford 693 mg of the desired crystalline Form I of ibandronate sodium.

### EXAMPLE 2: IBANDRONATE SODIUM CRYSTALLINE FORM I

Ibandronate sodium (800 mg) was charged into a clean and dry 4neck round bottom flask containing acetic acid (2.5 ml). The mixture was heated to 60-65°C and stirred for 15 minutes. The resultant solution was cooled to room temperature and ethylene glycol (15 ml) added. The solution was stirred at room temperature for 10-15 minutes. The separated solid was filtered and the solid was subjected to suction pressure for 30 minutes to afford 910 mg of the desired crystalline Form I of ibandronate sodium.

### EXAMPLE 3: IBANDRONATE SODIUM CRYSTALLINE FORM I

Ibandronate sodium (800 mg) was charged into a clean and dry 4neck round bottom flask containing formic acid (0.8 ml). The mixture was heated to 60-65°C and stirred for 15 minutes for clear solution. The resultant reaction solution was cooled to room temperature and ethylene glycol (10 ml) was added. The resultant suspension was stirred at room temperature for 15 minutes. The separated solid was filtered and the solid was subjected to suction pressure for 30 minutes, to afford 625 mg of the desired crystalline Form I of ibandronate sodium.

### EXAMPLE 4: IBANDRONATE SODIUM CRYSTALLINE FORM 1

Ibandronate sodium (602.1 mg) was charged into a clean and dry 4neck round bottom flask containing dimethylsulfoxide (DMSO; 2 ml). The mixture was heated to 150-160°C and stirred for 15 minutes for clear solution. The resultant homogenous reaction solution was cooled to room temperature and ethylene glycol (40 ml) was charged in lot wise (2x20 ml) and the resultant solution was stirred for 10-15 minutes. The separated solid was filtered and the solid was subjected to suction pressure for 30 minutes to afford 521 mg of the desired crystalline Form I of ibandronate sodium,

### EXAMPLE 5: PREPARATION OF IBANDRONATE SODIUM CRYSTALLINE FORM II

Ibandronate sodium (1 g) was charged into a clean and dry 4neck round bottom flask containing formamide (10 ml). The suspension was stirred at 50-60°C for 10-12 hrs. The solid was filtered and suck dried for 5 minutes to afford 454 mg of the desired crystalline Form II of ibandronate sodium.

### EXAMPLE 6: IBANDRONATE SODIUM CRYSTALLINE FORM II

Ibandronate sodium (1 g) was charged into a clean and dry round bottom flask containing formamide (10 ml). The obtained mixture was stirred at 0-5°C for 10-12 hrs. The solid was filtered and dried at 50-60°C for 2-3hrs to afford 502 mg of the desired crystalline Form II of ibandronate sodium.

### EXAMPLE 7: IBANDRONATE SODIUM CRYSTALLINE FORM II

Ibandronate sodium (1 g) was charged into a clean and dry 4 neck round bottom flask containing N, N-dimethyl acetamide (DMA; 10 ml). The reaction mixture was cooled to 0-5°C and stirred for 10-12hrs. The resultant reaction mixture was filtered and dried at 50-60°C for 2-3 hrs to afford 623 mg of desired crystalline Form II of ibandronate sodium.

### EXAMPLE 8: IBANDRONATE SODIUM CRYSTALLINE FORM II

Ibandronate sodium (1 g) was charged into a clean and dry round bottom flask containing N, N-dimethyl formamide (DMF; 10 ml). The mixture was cooled to 0-5°C and stirred for 10-12 hrs. The obtained solid was filtered and dried at 50-60°C for 2-3 hrs to afford 802 mg of desired crystalline Form II of ibandronate sodium.

### EXAMPLE 9: PREPARATION OF IBANDRONATE SODIUM CRYSTALLINE FORM III

Ibandronate sodium (800 mg) was charged into a clean and dry 4neck round bottom flask containing formamide (5 ml). The mixture was heated to 120-125°C and stirred for 15-30 minutes. To the resultant solution ethanol (25 ml) was added at 120-125°C and stirred for 30-60 minutes. The reaction solution was cooled to 25-35°C, and stirred for 2-3 hrs. The separated solid was filtered and the solid obtained was subjected to suction pressure for about 15 minutes to afford 1.16 g of desired crystalline Form III of ibandronate sodium.

### EXAMPLE 10: IBANDRONATE SODIUM CRYSTALLINE FORM III

Ibandronate sodium (1 g) was charged into a clean and dry 4neck round bottom flask containing formamide (5 ml). The mixture was heated to 120-125°C and stirred for 15 minutes. The resultant reaction solution was cooled to room temperature and n-propanol (25 ml) was added. The resultant solution was stirred for 1-2 hrs for solid separation. The separated solid was filtered and the solid obtained was dried at 60°C for 2 hours to afford 0.42 g of desired crystalline Form III of ibandronate sodium.

### EXAMPLE 11: IBANDRONATE SODIUM CRYSTALLINE FORM III

Ibandronate sodium (1 g) was charged into a clean and dry round bottom flask containing formamide (5 ml). The mixture was heated to 120-125°C followed by stirring for 15 minutes. The resultant solution was cooled to room temperature and n-butanol (25 ml) was charged slowly. The resultant reaction solution was stirred for 1-2 hrs up to solid separation. The separated solid was filtered and the solid obtained was dried at 50-60°C for 2-3 hr to afford 0.4 g of desired crystalline Form III of ibandronate sodium.

### EXAMPLE 12: PREPARATION OF IBANDRONATE SODIUM CRYSTALLINE FORM IV

Ibandronate sodium (602 mg) was charged into a clean and dry round bottom flask containing formic acid (0.6 ml). The mixture was heated to 60-70°c under stirring for 5-10 minutes. The solution was cooled to room temperature and ethyl methyl ketone (15 ml) was charged in one portion and stirred for 5-10 minutes. The separated solid was filtered and the solid obtained was subjected to suction pressure for 15 minutes to afford 464.2 mg of desired crystalline Form IV of ibandronate sodium.

### EXAMPLE 13: IBANDRONATE SODIUM CRYSTALLINE FORM IV

Ibandronate sodium (802 mg) was charged into a clean and dry round bottom flask containing formic acid (0.8 ml). The mixture was heated to 50-60°C and stirred for 5-10 minutes. To the resultant solution, n-propanol (10 ml) was added and stirred for 5-10 minutes. The separated solid was filtered and the solid obtained was subjected to suction pressure for 15 minutes to afford 914 mg of desired crystalline Form IV of ibandronate sodium.

### EXAMPLE 14: PREPARATION OF IBANDRONATE SODIUM CRYSTALLINE FORM V

Ibandronate sodium (600 mg) was charged into a clean and dry round bottom flask containing formic acid (0.6 ml). The mixture was heated to 60-65°C under stirring for 10-15 minutes. The resultant solution was cooled to 25-35°C followed by charging in of methyl tertiary butyl ether (MTBE; 15 ml). The resultant mixture solution was stirred for 15 minutes. The separated solid was filtered and the solid obtained was subjected to suction pressure for 15 minutes to afford 575 mg of desired crystalline Form V of ibandronate sodium having the X-ray powder diffraction pattern.

### EXAMPLE 15: IBANDRONATE SODIUM CRYSTALLINE FORM V

Ibandronate sodium (50 g) was taken into a clean and dry round bottom flask and a solution of formic acid and methyl tertiary butyl ether (1:1 ratio, 350 ml) was added. The mixture was stirred further to get clear solution. The undissolved material was removed by filtration. The filtrate was heated to 50°C under stirring for 15 minutes followed by addition of methyl tertiary butyl ether (500 ml). The resultant mixture was allowed to cool to 25°C and stirred for 15 minutes. The separated solid was filtered, washed with methyl tertiary butyl ether and then subjected to suction pressure for 15 minutes to afford the desired crystalline Form V of ibandronate sodium (60 g).

### EXAMPLE 16: IBANDRONATE SODIUM CRYSTALLINE FORM VI

Ibandronate sodium (1 g) was charged into a clean and dry round bottom flask containing N, N-dimethyl formamide (DMF; 10 ml), The mixture was heated to 50-60°C for 10-12 hours. The solid was filtered and suck dried to afford 1.51 g of desired Form VI of ibandronate sodium.

### EXAMPLE 17: IBANDRONATE SODIUM CRYSTALLINE FORM VII

Ibandronate sodium (800 mg) was charged into a clean and dry round bottom flask containing demineralized water (1 ml). The mixture was heated to 100°C and stirred for complete dissolution. The resultant solution was cooled to room temperature and isopropyl alcohol (20 ml) was added and stirred for 10-15 minutes. The separated solid was filtered and the solid was subjected to suction pressure for 15 minutes to afford 755 mg of desired crystalline Form VII of ibandronate sodium.

### EXAMPLE 18: PREPARATION OF IBANDRONATE SODIUM CRYSTALLINE FORM VIII

Ibandronate sodium (800 mg) was charged into a clean and dry round bottom flask containing formic acid (0.8 ml). The mixture was heated to 50-60°C and stirred for complete dissolution for 15 minutes followed by cooling the reaction solution to room temperature. Acetone (8 ml) was added into the solution and stirred for 10-15 minutes. The separated solid was filtered and the solid was subjected to suction pressure for 15 minutes to afford 720 mg of desired crystalline Form VIII of ibandronate sodium.

### EXAMPLE 19: IBANDRONATE SODIUM CRYSTALLINE FORM VIII

Ibandronate sodium (500 mg) was charged into a clean and dry round bottom flask containing demineralized water (2 ml). The mixture was heated to 60-65°C for complete dissolution. The resultant solution was evaporated slowly at room temperature to afford 485 mg of desired crystalline Form VIII of ibandronate sodium.

### EXAMPLE 20: PREPARATION OF IBANDRONATE SODIUM CRYSTALLINE FORM IX

Ibandronate sodium (500 mg) was charged into a clean and dry round bottom flask containing demineralized water (2 ml). The resultant clear solution was charged into a clean and dry petridish. The solution was freezed to -10°C in a freeze drying chamber, and dried under high vacuum (0.1 Torr) in the temperature range -10 to 10°C over a period of 7-8 hours to afford 420 mg of the desired Form IX of ibandronate sodium.

### EXAMPLE 21: PREPARATION OF IBANDRONATE SODIUM CRYSTALLINE FORM X

Ibandronate sodium (802 mg) was charged into a clean and dry round bottom flask containing demineralized water (3 ml). The mixture was heated to 100°C and stirred for complete dissolution, To the resultant solution n-propyl alcohol (20 ml) was added at 100°C and stirred for 5-10 minutes. The separated solid was filtered and the solid was subjected to suction pressure for 15 minutes to afford 836 mg of desired crystalline Form X of ibandronate sodium.

### EXAMPLE 22: PREPARATION OF IBANDRONATE SODIUM CRYSTALLINE FORM XI

Ibandronate sodium (803.2 mg) was charged into a clean and dry round bottom flask containing acetic acid (2.5 ml). The mixture was heated to 60-65°C and stirred for complete dissolution. To the resultant solution IPA (2x20 ml) was added in lot wise and stirred for 15 minutes for solid separation. The separated solid was filtered and the solid was subjected to suction pressure for 15 minutes to afford 954 mg of desired crystalline Form XI of ibandronate sodium.

### EXAMPLE 23: PREPARATION OF IBANDRONATE SODIUM CRYSTALLINE FORM XII

Ibandronate sodium (500 mg) was charged into a clean dry round bottom flask containing formic acid (1.5 ml). The mixture was heated to 60-65°C-under stirring for complete dissolution. The resultant solution was kept aside for slow evaporation under ambient conditions to afford 477 mg of desired crystalline Form XII of ibandronate sodium.

### EXAMPLE 24: PREPARATION OF IBANDRONATE SODIUM CRYSTALLINE FORM XIII

Ibandronate sodium (800 mg) was charged into a clean and dry round bottom flask containing formic acid (0.8 ml). The mixture was heated to 50-60°C and stirred for complete dissolution followed by cooling the solution to room temperature. To the resultant reaction solution acetonitrile (8 ml) was added at room temperature for 15-20 minutes. The separated solid was filtered and suck dried for 10-15 minutes to afford 655 mg of desired form XIII of ibandronate sodium.

### EXAMPLE 25: PREPARATION OF IBANDRONATE SODIUM CRYSTALLINE FORM XIV

Ibandronate sodium (800 mg) was charged into a clean and dry round bottom flask containing formic acid (0.8 ml). The mixture was heated to 60-65°C and stirred for complete dissolution followed by cooling the reaction solution to room temperature. To the resultant reaction solution 1, 4 dioxane (2x10 ml) was added lot wise and stirred at room temperature for 15 minutes. The separated solid was filtered and suck dried for 15 minutes to afford 720 mg of desired crystalline Form XIV of ibandronate sodium.

### EXAMPLE 26 PREPARATION OF IBANDRONATE SODIUM CRYSTALLINE FORM XV

Ibandronate sodium (600 mg) was charged into a clean and dry round bottom flask containing formic acid (0.6 ml). The mixture was heated to 60-65°C under stirring for 15 minutes. The reaction solution was cooled to room temperature. To the resultant solution dichloromethane (10+5 ml) was charged in lotwise and stirred for room temperature for 15 minutes. The separated solid was filtered and the solid was subjected to suction pressure for 15 minutes to afford 350 mg of desired crystalline Form XV of ibandronate sodium.

### EXAMPLE 27: PREPARATION OF IBANDRONATE SODIUM CRYSTALLINE FORM XVI

Ibandronate sodium (1.02 g) was charged into a clean and dry round bottom flask containing ml of dimethylacetamide (DMA; 8 ml). The resultant mixture was stirred at 50-60°C for 10-12 hours. The solid was filtered and subjected to suction pressure for 15 minutes to afford 910 mg of desired crystalline Form XVI of ibandronate sodium.

### EXAMPLE 28: PREPARATION OF IBANDRONATE SODIUM CRYSTALLINE FORM XVII

Ibandronate sodium (1.01 g) was charged into a clean dry round bottom flask containing dimethylsulfoxide (DMSO; 8 ml). The resultant mixture was stirred at room temperature for 10-12 hours. The solid was filtered and subjected to suction pressure for 15 minutes to afford 900 mg of desired crystalline Form XVII of ibandronate sodium.

### EXAMPLE 29: PREPARATION OF IBANDRONATE SODIUM CRYSTALLINE FORM XVIII:

Ibandronate sodium (1.02 g) was charged into a clean and dry round bottom flask containing dimethyl acetamide (DMA; 8 ml). The reaction mixture was stirred at room temperature for 10-12 hours. The solid was filtered and the solid obtained was subjected to suction pressure for 15 minutes to afford 850 mg of desired crystalline Form XVIII of ibandronate sodium.

### EXAMPLE 30: PREPARATION OF IBANDRONATE SODIUM CRYSTALLINE FORM XIX:

Ibandronate sodium (1.01 g) was charged into a clean and dry round bottom flask containing N, N-dimethylformamide (DMF; 6 ml). The reaction mixture was stirred at room temperature for 10-12 hours. The solid was filtered and subjected to suction pressure for 15 minutes to afford 955 mg of desired crystalline Form XIX of ibandronate sodium.

### EXAMPLE 31: PREPARATION OF IBANDRONATE SODIUM CRYSTALLINE FORM XX:

Ibandronate sodium (1 g) was charged into a clean and dry round bottom flask containing DM water (1.5 ml). The reaction mixture was cooled to 0-5°C under stirring for 10-12 hours. The solid was filtered and subjected to suction pressure for 15 minutes to afford 159 mg of desired crystalline Form XX of ibandronate sodium.

### EXAMPLE 32: PREPARATION OF IBANDRONATE SODIUM CRYSTALLINE FORM XXI:

Ibandronate sodium (1.01 g) was charged into a clean and dry round bottom flask containing dimethylsulfoxide (DMSO; 8 ml). The reaction mixture was heated to 50-60°C and stirred for 10-12 hours. The solid was filtered and subjected to suction pressure for 15 minutes to afford 880 mg of desired crystalline Form XXI of ibandronate sodium.

### EXAMPLE 33: PREPARATION OF IBANDRONATE SODIUM CRYSTALLINE FORM XXII

Ibandronate sodium (1 g) was charged into a clean and dry round bottom flask containing formamide (2 ml). The reaction mixture was heated to 155-160°C and stirred for 15 minutes. The reaction solution was cooled to room temperature. To the resultant reaction solution n-butanol (10 ml) was added and stirred for 15 minutes. The separated solid was filtered and subjected to suction pressure and dried under vacuum at room temperature for 2-3 hrs to afford 800 mg of desired crystalline Form XXII of ibandronate sodium.

### EXAMPLE 34: PREPARATION OF IBANDRONATE SODIUM CRYSTALLINE FORM XXIII:

Ibandronate sodium (802.1 mg) was charged into a clean and dry 4neck round bottom flask containing formic acid (0.8 ml). The reaction mixture was heated to 50-60°C and stirred for 5-10 minutes. To the resultant solution n-propanol (10 ml) was added under stirring at 50-60°C for 5-10 minutes. The separated solid was filtered and subjected to suction pressure for 15 minutes to afford 914 mg of desired crystalline Form XXIII of ibandronate sodium.

### EXAMPLE 35: PREPARATION OF IBANDRONATE SODIUM CRYSTALLINE FORM XXIV:

Ibandronate sodium (603.1 mg) was charged into a clean and dry round bottom flask containing dimethylsulfoxide (DMSO; 2 ml). The reaction mixture was heated to 150-160°C and stirring for 15 minutes. The resultant reaction solution was cooled to room temperature. To the resultant solution ethyl acetate (20 ml) was added and stirred for 15 minutes. The separated solid was filtered and subjected to suction pressure for 15 minutes to afford 575 mg of desired crystalline Form XXIV of ibandronate sodium.

### EXAMPLE 36: PREPARATION OF IBANDRONATE SODIUM CRYSTALLINE FORM XXV:

Ibandronate sodium (800 mg) was dissolved in formamide (5 ml) while heating to a temperature of 120 °C. To the solution n-butanol (25 ml) was added and stirred for a period of 1 hour at 120°C. The suspension was cooled to a room temperature and stirred for 75 minutes and then filtered followed by suck drying under vacuum to obtain 712 mg of title compound.

### EXAMPLE 37: PREPARATION OF IBANDRONATE SODIUM CRYSTALLINE FORM XXVI:

Ibandronate sodium (602.2 mg) was dissolved in dimethylsulfoxide (DMSO; 2 ml) while heating to a temperature of 150 to 160°C and then the obtained solution was cooled to room temperature. To the resultant solution methyl tertiary butyl ether (MTBE; 20 ml) was added and stirred for 1 to 2 hours at a room temperature. The suspension was filtered and suck dried under vacuum to obtain 587 mg of title compound.

### EXAMPLE 38: PREPARATION OF IBANDRONATE SODIUM CRYSTALLINE FORM XXVII:

Ibandronate sodium (790 mg) was charged into a clean and dry round bottom flask containing water (3 ml). The reaction mixture was heated to 100°C and stirred for 15 minutes and check for complete dissolution. THF (35 ml) was added to the reaction solution at 100°C and stirred for 5-10 minutes. The separated solid was filtered and subjected to suction pressure for 15 minutes to afford 986.2 mg of desired crystalline Form XXVII of ibandronate sodium.

### EXAMPLE 39: PREPARATION OF IBANDRONATE SODIUM CRYSTALLINE FORM XXVIII:

Ibandronate sodium (800 mg) was charged into a clean and dry round bottom flask containing acetic acid (2.5 ml). The reaction mixture to 60-65°C followed by stirring for 15 minutes and check for complete dissolution. Ethyl methyl ketone (30 ml) was added to the reaction solution at 60-65°C and stirred for 5-10 minutes. The separated solid was filtered and subjected to suction pressure for 15 minutes to afford 725 mg of desired crystalline Form XXVIII of ibandronate sodium.

### EXAMPLE 40: PREPARATION OF IBANDRONATE SODIUM CRYSTALLINE FORM XXIX:

Ibandronate sodium (1 g) was charged into a clean and dry round bottom flask containing formic acid (2.5 ml). The reaction mixture to 50-60°C and stirred for 10-12 hours. The solid was filtered and suck dried for 15 minutes under vacuum to afford 393.2 mg of desired crystalline Form XXIX of ibandronate sodium.

### EXAMPLE 41: PREPARATION OF IBANDRONATE SODIUM CRYSTALLINE FORM XXX:

Ibandronate sodium (1 g) was charged into a clean and dry round bottom flask containing acetic acid (1 ml). The reaction mixture to 50-60°C and stirred for 10-12 hours. The solid was filtered and suck dried for 15 minutes under vacuum to afford 868.2 mg of desired crystalline Form XXX of ibandronate sodium.

### EXAMPLE 42: PREPARATION OF IBANDRONATE SODIUM CRYSTALLINE FORM XXXI:

Ibandronate sodium (500 mg) was charged into a clean and dry round bottom flask containing acetic acid (2.5 ml). The reaction mixture was heated to 60-65°C and stirred for 10-15 minutes. The solution was slowly evaporated at room temperature to afford desired crystalline Form XXXI of ibandronate sodium,

### EXAMPLE 43: PREPARATION OF IBANDRONATE SODIUM CRYSTALLINE FORM ALPHA:

Ibandronate sodium formic acid solvate (Form V) (60 g) was heated in hot air oven at a temperature of 100°C for nine hours at atmospheric pressure to obtain the ibandronate sodium Form Alpha.

Yield: 50 g; Moisture content: 0.66 % (KF method) and Formic acid content 0.16%w/w.

### EXAMPLE 44: PREPARATION OF IBANDRONATE SODIUM CRYSTALLINE FORM BETA

Ibandronate sodium formic acid solvate (Form V) (50 g) was heated in hot air oven at temperature of 100°C for eight hours at atmospheric pressure. The material was removed from the oven and exposed to atmospheric conditions for 24 hours to obtain the ibandronate sodium Form Beta.

Yield: 45 g; Moisture content: 8.9% (KF method); Formic acid content 0.52%w/w.

DSC thermogram: Shows two peaks- Peak -01 at 127.3°C; Peak -02 at 183.8°C.

### EXAMPLE 45: PREPARATION OF IBANDRONATE SODIUM CRYSTALLINE FORM BETA

The Form Alpha of ibandronate sodium obtained in example 2 (8 g) was exposed to humid atmosphere (having relative humidity in the range of 70-80%) in fluid bed dryer at a temperature in the range between 25°C to 35°C for 24 hours to obtain Form Beta.

Yield: 8 g; Moisture content: 9.2% (KF method).

### EXAMPLE 46: PREPARATION OF IBANDRONATE SODIUM CRYSTALLINE FORM BETA

Ibandronate sodium (50 g) was taken into a clean and dry round bottom flask and a solution of formic acid and methyl tertiary butyl ether (1:1 ratio, 350 ml) was added. The mixture was heated to 40 to 45°C to get clear solution. The undissolved material was removed by filtration. The filtrate was heated to 45 to 50°C under stirring for 10 minutes followed by addition of methyl tertiary butyl ether (500 ml). The resultant mixture was allowed to cool to room temperature and stirred for 20 minutes. The separated solid was filtered, washed with methyl tertiary butyl ether (100 ml) and then subjected to suction pressure for 15 minutes. The compound further dried at 50°C under vacuum for a period of 3 hrs to afford crystalline Form V of ibandronate sodium.

M.C.: 2.37 % by KF method; Formic acid content: 15.25 % w/w

Crystalline Form V of ibandronate sodium (65 g), obtained from above process, was heated in hot air oven at a temperature of 100°C for 15 hrs to obtain ibandronate sodium Form Alpha.

Yield: 39 g; Moisture content: 2.97 % (KF method) and Formic acid content 0.16%w/w.

The above-obtained material (5 g) was removed from the oven and exposed to humid atmosphere (having relative humidity of 60 % at 25°C) in humidification chamber for 30 to 40 minutes to obtain Form Beta.

Yield. 6.3 g; Moisture content: 9.02 % (KF method); Formic acid content 0.18 %w/w.

There have been disclosed hereinbefore the crystalline forms, crystalline solvates, and methods defined by the following numbered paragraphs:
1. A crystalline form of ibandronate sodium selected from the group consisting of Form I, Form II, Form III, Form IV, Form V, Form VI, Form VII, Form VIII, Form IX, Form X, Form XI, Form XII, Form XIII, Form XIV, Form XV, Form XVI, Form XVII, Form XVIII, Form XIX, Form XX, Form XXI, Form XXII, Form XXIII, Form XXIV, Form XXV, Form XXVI, Form XXVII, Form XXVIII, Form XXIX, Form XXX, Form XXXI, Form Alpha and Form Beta.
2. The crystalline form of ibandronate sodium of paragraph 1 , selected from the group consisting of Form I, Form V, Form VIII, Form Alpha and Form Beta.
3. The crystalline Form I of ibandronate sodium of paragraph 2 comprising an X-ray powder diffraction pattern with characteristic peaks at about 5.2, 17.4, 20.1 , 25.2, and 31.3 + 0.2 degrees two theta.
4. A crystalline Form I comprising an X-ray powder diffraction pattern substantially as depicted in Figure 1.
5. The crystalline Form V of ibandronate sodium of paragraph 2 comprising an X-ray powder diffraction pattern with characteristic peaks at about 4.8,10.8, 19.7, 22.0, and 31.1 + 0.2 degrees two theta.
6. A crystalline Form V comprising an X-ray powder diffraction pattern substantially as depicted in Figure 5.
7. The crystalline Form VIII of ibandronate sodium of paragraph 2 comprising an X-ray powder diffraction pattern with characteristic peaks at about 6.1 , 16.7, 18.1 , 20.3, and 30.2 + 0.2 degrees two theta.
8. A crystalline Form VIII comprising an X-ray powder diffraction pattern substantially as depicted in Figure 8.
9. A crystalline solvate of ibandronate sodium.
10. The crystalline solvate of ibandronate solvate of paragraph 9 wherein said solvate is water, hydrocarbon solvents, ketones, alcohols, nitriles, ethers, amines, esters and acids
11. The crystalline solvate of ibandronate sodium of paragraph 9 wherein said solvate is n-hexane, n-heptane, cyclohexane, toluene, xylene, acetone, n- butanone, methyl isobutyl ketone, ethyl methyl ketone, acetone, acetonitrile, propionitrile, methanol, ethanol, isopropyl alcohol, n-propanol, t-butyl alcohol, n-butanol, sec- butanol, dimethyl sulfoxide, methyl tertiary butyl ether, dichloromethane, formamide, dimethyl ether, diethyl ether, diisopropyl ether, tetrahydrofuran, acetic acid, formic acid, citric acid, succinic acid, ethyl acetate, dimethylformamide, or dimethylacetamide.
12. A method of making ibandronate sodium Form Alpha comprising desolvating an ibandronate sodium solvate such that the residual solvent content is about 1 % w/w or less.
13. The method of paragraph 12 wherein said ibandronate sodium solvate is selected from the group consisting of Form I, Form V and Form VIII.
14. The method of paragraph 13 wherein said ibandronate sodium solvate is desolvated at a temperature of between about 50 and 150 degrees C.
15. A method of making ibandronate sodium Form Beta comprising exposing ibandronate sodium Form Alpha to a humid environment for a time sufficient to convert at least a portion of said ibandronate sodium Form Alpha to ibandronate sodium From beta.
16. The method of paragraph 15 wherein said humid environment has a humidity of more than 30% at a temperature of between about 20 and about 80 degrees C.
17. The Crystalline Form Alpha of ibandronate sodium of paragraph 2 comprising an X-ray powder diffraction pattern with characteristic peaks at about 5.5, 6.3, 19.3, and 23.1 +-0.2 degrees two theta.
18. A crystalline Form Alpha comprising an X-ray powder diffraction pattern substantially as depicted in Figure 32.
19. The crystalline Form Beta of ibandronate sodium of paragraph 2 comprising an X-ray powder diffraction pattern with characteristic peaks at about 5.5, 10.9, 19.3, 23.1 and 33.2 +-0.2 degrees two theta.
20. A crystalline Form Beta comprising an X-ray powder diffraction pattern substantially as depicted in Figure 33.

## Claims

1. A crystalline form of ibandronate sodium which is Form Alpha.

2. A method of making ibandronate sodium Form Alpha comprising desolvating an ibandronate sodium solvate such that the residual solvent content is about 1% w/w or less.

3. The method of claim 2 wherein said ibandronate sodium solvate is selected from the group consisting of Form I, Form V and Form VIII.

4. The method of claim 3 wherein said ibandronate sodium solvate is desolated at a temperature of between about 50 and 150 degrees C.

5. The crystalline Form Alpha of ibandronate sodium of claim 1 having an X-ray powder diffraction pattern with characteristic peaks at about 5.5, 6.3, 19.3, and 23.1 ± 0.2 degrees two theta.

6. A crystalline Form Alpha of ibandronate sodium of claim 1 having an X-ray powder diffraction pattern substantially as depicted in Figure 32.

7. The crystalline Form Alpha of ibandronate sodium of claim 1 having an X-ray powder diffraction pattern with characteristic peaks at about 11.0, 15.0, 18.4, 21.0, 22,4, 28.1, and 33.3 ± 0.2 degrees.

8. A crystalline Form Alpha of ibandronate sodium of claim 1 with an DSC pattern substantially as depicted in Figure 39.

9. A method according to claim 2 wherein said ibandronate sodium solvate is a formic acid solvate, acetic acid solvate, ethylene glycol solvate, or DMSO solvate.

10. A method according to claim 2 wherein said ibandronate sodium solvate is sodium formic acid solvate Form V and is desolvated at a temperature of between about 50°C and about 150°C.
